# EUROPEAN PATENT APPLICATION

(11) **EP 4 364 567 A1**
(43) Date of publication of application: **08.05.2024**
(21) Application number: 22833576.6
(22) Date of filing: 28.06.2022
(51) Int. Cl.: A01N 33/08, A01N 43/14, A01N 33/16, A01N 43/40, A01N 47/44, A61K 8/41, A61K 8/49, A61K 8/43, A61Q 5/02, A61Q 19/00

(54) **ANTISEPTIC, ANTIBACTERIAL, AND PRESERVATIVE COMPOSITION**

(30) Priority: 29.06.2021 KR 20210084966; 29.06.2021 KR 20210084967; 29.06.2021 KR 20210084968; 24.06.2022 KR 20220077425
(71) Applicant: Bjbiochem Co., Ltd., Yuseong-gu, Daejeon 34054 (KR)
(72) Inventor: JEONG, Gug In, Daejeon 34021 (KR); RYU, Jae Chun, Daejeon 35210 (KR); CHA, Kyung On, Daejeon 34050 (KR); KIM, Na Rae, Daejeon 34070 (KR); WON, Chae Hoon, Daejeon 34008 (KR)
(74) Representative: AWA Sweden AB
(86) International application number: PCT/KR2022/009221
(87) International publication number: WO 2023/277520

(57) **Abstract**

Disclosed is an antimicrobial and preservative composition to prevent decay and maintain physical properties of water-containing products used as cleaning or fragrance products for clothing, dishes and living spaces, wet wipes, cosmetics, and the like, and products containing the composition such as cosmetics, sanitary products, and household chemical products. The antimicrobial and preservative composition contains a compound represented by Formula 1 and a compound represented by Formula 2, Formula 3, Formula 4 or Formula 5, thereby solving problems such as harmfulness to the human body and skin irritation, while improving antimicrobial activity.

## Description

### [Technical Field]

The present invention relates to an antimicrobial and preservative composition for preventing decay and maintaining physical properties of water-containing products or water-free products (cosmetic packs) used as cleaning or fragrance products for clothing, dishes and living spaces, wet wipes, shampoo, cosmetics (cream, lipsticks, etc.), and the like, and products containing the antimicrobial and preservative composition.

### [Background Art]

All products containing water require antimicrobial agents because microorganisms readily propagate in water. Antimicrobial agents are agents that inhibit the growth of or kill microorganisms such as bacteria.

In particular, detergent products containing a great amount of water necessarily contain preservatives for preventing the proliferation and decay of microorganisms and antimicrobial agents for removing contaminant microorganisms in order to maintain quality thereof for a long time. These products require antimicrobial components because they have a very long shelf life, are very likely to come into contact with microorganisms during use and storage thereof, are blended with various carbon and nitrogen sources, and contain a high amount of water essential for microbial growth.

Recently, the harmfulness of antimicrobial agents to the human body has been controversial. However, if antimicrobial agents are not used in products or the antimicrobial property thereof is weak, there is a high risk of product deterioration and pathogen transmission, and microbial metabolism causes skin troubles and diseases. Therefore, antimicrobial agents are inevitably used.

Isothiazolinone ingredients such as methylisothiazolinone, chloromethylisothiazolinone, and 1,2-benzisothiazol-3(2H)-one, which are toxic chemicals, are still used as preservatives in clothes, dishes, household cleaning or fragrance products, wet wipes, shampoo, cosmetics (creams, lipstick, etc.) and the like.

The isothiazolinone ingredients, which are toxic chemicals, are most widely used due to economic efficiency and potent effects thereof, but are being increasingly regulated because they may act as carcinogens.

Recently, regulations for respective product groups have been strengthened in Korea as antimicrobial agents may affect public health. The movement to strengthen antimicrobial regulations is accelerating. For example, related laws and regulations are strengthened, such as wet wipes are classified into other sanitary products under the Sanitary Product Management Act, and the Ministry of Food and Drug Safety released a notice associated with usable raw materials for kitchen detergents. However, due to inappropriate use of antimicrobial agents, the application of highly safe and excellent antimicrobial agents is being narrowed.

Therefore, there is a demand for an antimicrobial agent that is safely used in products including cosmetics, sanitary products, and household chemical products and exhibits excellent antimicrobial activity.

### [Prior art]

### [Patent Document]

(Patent Document 1) Korean Patent No. 10-2233825

### [Disclosure]

### [Technical Problem]

Accordingly, during research and development of a method for regulating microorganisms in a different manner from conventional antimicrobial agents that harm the human body in various ways, the present inventors found that a combination of a compound having a surfactant structure represented by Formula 1 with a preservative compound represented by Formula 2, Formula 3, Formula 4 or Formula 5 enables the microbial control material to be easily incorporated into the product formulation, and provides synergistic antimicrobial, disinfecting and antiseptic effects compared to each of the compounds described above and also considered that the combination has the effect of offsetting the decline in antimicrobial, disinfecting and preservative functions caused by the ionic nature of the product base, and thus is more effectively capable of controlling microorganisms, thus completing the present invention.

It is one object of the present invention to provide an antimicrobial and preservative composition containing a compound represented by Formula 1 and a compound represented by Formula 2, Formula 3, Formula 4 or Formula 5.

It is another object of the present invention to provide various types of products including the antimicrobial and preservative composition.

It is another object of the present invention to provide an antimicrobial method for treating bacteria with the antimicrobial and preservative composition.

### [Technical Solution]

The present invention discloses the following solutions to the above problems.

In accordance with one aspect of the present invention, provided is an antimicrobial and preservative composition containing (a) a compound represented by Formula 1 and (b) a compound represented by Formula 2, Formula 3, Formula 4 or Formula 5.

The antimicrobial and preservative composition of the present invention is advantageously capable of controlling a wide range of microorganisms and of solving problems such as harmfulness to the human body and skin irritation caused by preservatives.

In accordance with another aspect of the present invention, provided is a product including the antimicrobial and preservative composition.

In accordance with another aspect of the present invention, provided is an antimicrobial method including treating bacteria with the antimicrobial and preservative composition.

### [Advantageous Effects]

The combination of the ingredients discovered by the present inventors exerts synergistic antimicrobial effects.

In addition, various products such as cosmetics, sanitary products, and household chemical products including the antimicrobial and preservative composition discovered by the present inventors enable emulsification and solubilization suitable for the characteristics of surfactants and exhibit excellent antimicrobial activity and a broad antimicrobial spectrum.

In addition, the antimicrobial and preservative composition of the present invention exhibits a wide range of efficacy in compositions based on anionic and nonionic surfactants and compositions based on cationic surfactants, thus being utilized in a wide range of applications.

The effects of the present invention are not limited to those described above and various effects may be derived from the following description within the scope of those skilled in the art.

### [Best Mode]

Hereinafter, embodiments of the present invention will be described in more detail.

The detailed description will be given below. Meanwhile, each description and embodiment disclosed herein may also be applied to other descriptions and embodiments, respectively. That is, all combinations of various elements disclosed herein fall within the scope of the present invention. Also, the detailed description described below should be not construed as limiting the scope of the present invention.

Terms, such as "comprising", used herein should be considered as open-ended terms that may include other embodiments, unless specifically mentioned otherwise in the phrases or sentences including the term.

Terms and words used in the description and the claims are not construed as being limited to conventional meanings in dictionaries, but are construed to have meanings and concepts corresponding to the spirit and scope of the present invention based on a principle that the inventor can properly define concepts of the terms in order to explain the invention in the best way.

### Antimicrobial and preservative composition

The present invention provides an antimicrobial and preservative composition containing (a) a compound having a represented by Formula 1 and (b) a compound represented by Formula 2, Formula 3, Formula 4 or Formula 5: 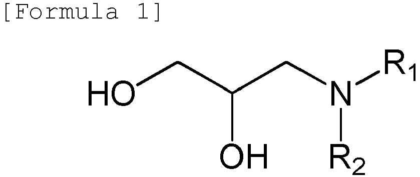 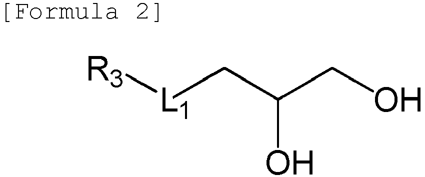 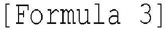 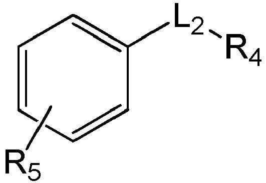 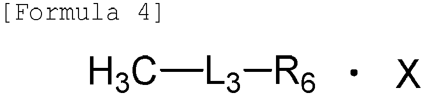 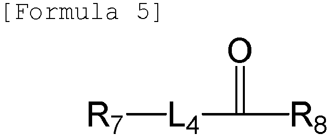 wherein
in Formula 1,
R₁ is hydrogen, and
R₂ is substituted or unsubstituted C₆-C₁₈ alkyl, or substituted or unsubstituted C₆-C₁₈ alkenyl,
in Formula 2,
L₁ is -CH₂-, -O-, -C(=O)- or -O-C(=O)-, and
R₃ is substituted or unsubstituted C₁-C₁₅ alkyl, or substituted or unsubstituted C₂-C₁₅ alkenyl,
in Formula 3,
L₂ is -(CH=CH)-, -(CH₂)ₘ-, -C=O- or -O-,
R₄ is -C(=O)-Rₐ, -OH, -O⁻Na⁺, -(CH₂)ₙ-OH, substituted or unsubstituted C₁-C₁₅ alkyl, or substituted or unsubstituted C₂-C₁₅ alkenyl,
R₅ is hydrogen, halogen, -OH, -OR_{b} or -(C=O)-O-R_{c}, and
Rₐ to R_{c} are each independently hydrogen, substituted or unsubstituted C₁-C₁₅ alkyl, or substituted or unsubstituted C₂-C₁₅ alkenyl,
wherein m and n are each independently an integer of 1 to 5,
in Formula 4,
L₃ is -C(=O)-, -(CH=CH)-(CH₂)ₒ-, -C(CH₃)₂-CH₂-CH(CH₃)-CH₂- or -(CH₂)ₚ-,
wherein o and p are each independently an integer of 1 to 15, and
- (CH₂)ₚ- is substituted with C₁-C₁₅ alkyl,
R₆ is 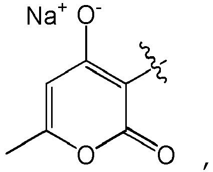 -C(=O)-NH-OH, 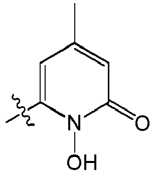 or 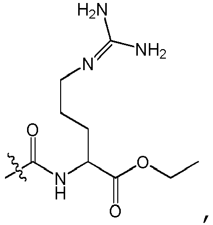 and
X is NH₂-CH₂-CH₂-OH or nothing (null), and
in Formula 5,
R₇ is substituted or unsubstituted C₁-C₁₅ alkyl, -COOH, -OR_{d} or -COO⁻Na⁺,
R₈ is substituted or unsubstituted C₁-C₁₅ alkyl, -OH or -O⁻Na⁺,
L₄ is substituted or unsubstituted -(CH₂)_{q}-, substituted or unsubstituted -(CH₂)ᵣ-N(Rₑ)-(CH₂)ₛ-, or substituted or unsubstituted phenylene(-C₆H₄-),
R_{d} is substituted or unsubstituted C₁-C₁₅ alkyl, and
Rₑ is substituted or unsubstituted C₁-C₁₅ alkyl or - (CH₂)ₜ-N[(CH₂)ᵤ-CO₂⁻)]₂,
wherein q, r, s, t, and u are each independently an integer of 1 to 5.

As used herein, the term "antimicrobial and preservative composition" refers to an antiseptic composition, may encompass all additives that may be used to prevent deterioration, decay, discoloration, and chemical change of the composition to which it is added, and may also encompass functional antibiotics for suppressing the proliferation of microorganisms such as bacteria, molds, and yeasts to inhibit the growth of or kill decaying microorganisms.

In the present invention, the antimicrobial and preservative composition may have antibacterial or antifungal activity against bacteria or fungi, but is not limited thereto.

As used herein, the term "antibacterial" refers to an action of inhibiting or controlling the growth and proliferation of bacteria.

As used herein, the term "antifungal" means an action of inhibiting or controlling the growth and proliferation of fungi.

In the present invention, the antimicrobial and preservative composition may have antibacterial activity against *Escherichia coli, Pseudomonas aeruginosa, Staphylococcus aureus, Klebsiella pneumoniae,* MRSA (*methicillin-resistant Staphylococcus aureus*), *Bacillus subtilis, Vibrio parahaemolyticus, Salmonella typhimurium, Listeria monocytogenes* and *Shigella flexneri,* and have antifungal activity against *Candida albicans,* and *Aspergillus niger,* but is not limited thereto.

In the present invention, the compounds represented by Formulas 2 to 5 are preservative compounds. The term used herein "preservative" refers to a chemical substance that is added to regulate the quality of industrially produced and distributed processed food, beverages, cosmetics, medicines, sanitary products, household chemicals, and the like, and is used to prevent deterioration of quality of products due to decay by microorganisms or decomposition of chemical components. That is, the preservative exhibits antibacterial activity against at least one microorganism of bacteria, molds, and yeasts, and thus is used as an antimicrobial, antibacterial and/or antifungal agent.

In the present invention, the antimicrobial and preservative composition may be used to control at least one microorganism selected from the group consisting of *Escherichia coli, Pseudomonas aeruginosa, Staphylococcus aureus, Klebsiella pneumoniae,* MRSA (*methicillin-resistant Staphylococcus aureus*), *Bacillus subtilis, Vibrio parahaemolyticus, Salmonella typhimurium, Listeria monocytogenes, Shigella flexneri, Candida albicans,* and *Aspergillus niger,* but is not limited thereto.

In the present invention, the compound represented by Formula 1 and the compound represented by Formula 2, Formula 3, Formula 4 or Formula 5 may be present in a weight ratio of 0.01:9.99 to 9.99:0.01, specifically the compound represented by Formula 1 and the compound represented by Formula 2, Formula 3, Formula 4 or Formula 5 may be present in a weight ratio of 6:4, 7:3 and/or 8:2, but the present invention is not limited thereto.

Specifically, the compound represented by Formula 1 and the compound represented by Formula 2, Formula 3, Formula 4 or Formula 5 are present in a content ratio within the range defined above, thereby advantageously improving the microbial control effect.

In the present invention, the compound represented by Formula 1 is 3-(dodecylamino)propane-1,2-diol, which is a compound represented by Formula 1-1, or 3-(tetradecylamino)propane-1,2-diol represented by Formula 1-2, but is not limited thereto.

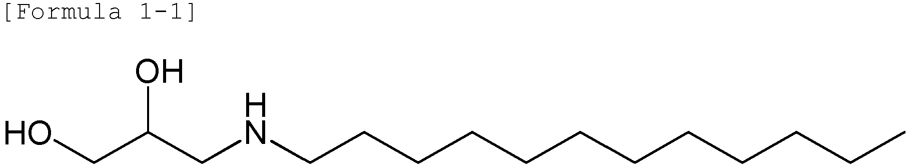

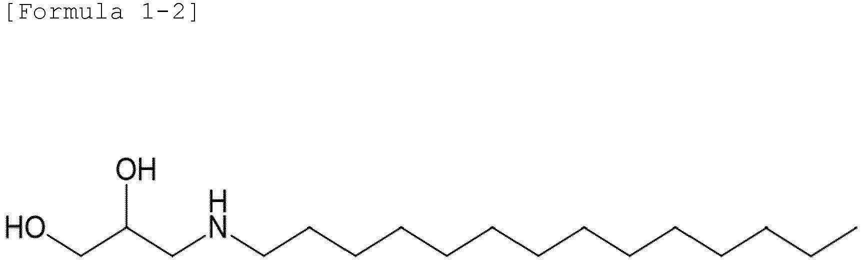

Specifically, the compound represented by Formula 1-1 or the compound represented by Formula 1-2 exhibits excellent emulsification and solubilization effects and is mixed with a compound represented by Formula 2, Formula 3, Formula 4, or Formula 5 to produce an antimicrobial composition, which is capable of inhibiting microorganisms such as *Escherichia coli, Pseudomonas aeruginosa, Staphylococcus aureus, Klebsiella pneumoniae, MRSA (methicillin-resistant Staphylococcus aureus), Bacillus subtilis, Vibrio parahaemolyticus, Salmonella typhimurium, Listeria monocytogenes, Shigella flexneri, Candida albicans* and *Aspergillus niger,* and thus exhibits antibacterial and antifungal properties against these microorganisms, and is advantageously applicable to various products such as liquid and solid cosmetics, sanitary products, and household chemical products.

In the present invention, in Formula 2, L₁ is -CH₂-, - O- or -O-C(=O)-, and R₃ is substituted or unsubstituted C₂-C₁₀ alkyl, but is not limited thereto.

Specifically, in Formula 2, L₁ is -CH₂-, and R₃ is substituted or unsubstituted C₂-C₈ alkyl, but is not limited thereto.

Also, in Formula 2, L₁ is -O-, and R₃ is substituted or unsubstituted C₃-C₉ alkyl, but is not limited thereto.

Also, in Formula 2, L₁ is -O-C(=O)- and R₃ is substituted or unsubstituted C₈-C₁₀ alkyl, but is not limited thereto.

In the present invention, the compound represented by Formula 2 includes at least one selected from the group consisting of hexane-1,2-diol, octane-1,2-diol, decane-1,2-diol, hexyl glycerin, octyl glycerin, ethylhexylglycerin, and glyceryl monocaprylate, but is not limited thereto.

Specifically, the hexane-1,2-diol has a structure of 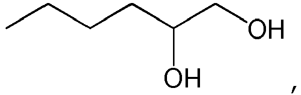 the octane-1,2-diol has a structure of 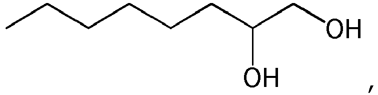 the decane-1,2-diol has a structure of 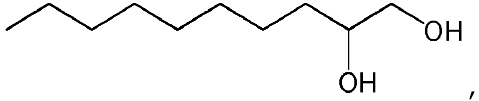 the hexyl glycerin has a structure of 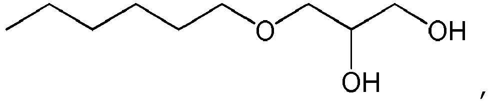 the octyl glycerin has a structure of 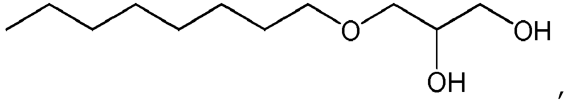 the ethylhexylglycerin has a structure of 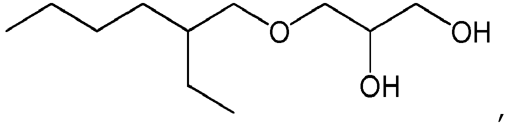 and the glyceryl monocaprylate has a structure of

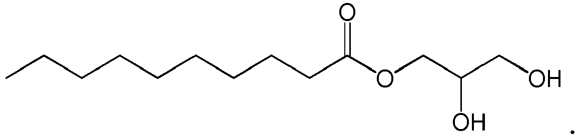

In the present invention, in the compound represented by Formula 3, L₂ is - (CH=CH) -, -(CH₂)ₘ-, -C=O- or -O-, R₄ is - C(=O)-Rₐ, -OH, -O⁻Na⁺, -(CH₂)ₙ-OH, or substituted or unsubstituted C₁-C₆ alkyl, and R₅ is hydrogen, -OH or -OR_{b}, wherein Rₐ and R_{b} are each independently hydrogen, substituted or unsubstituted C₁-C₆ alkyl, and m and n are each independently an integer of 2 to 4, but is not limited thereto.

Specifically, in the compound represented by Formula 3, L₂ is - (CH=CH) -, R₄ is -C(=O)-Rₐ, wherein Rₐ is hydrogen or substituted or unsubstituted C₁-C₃ alkyl, and R₅ is hydrogen, -OH or -OR_{b}, wherein R_{b} is hydrogen or substituted or unsubstituted C₁-C₃ alkyl, but is not limited thereto.

Also, in the compound represented by Formula 3, L₂ is - (CH₂)ₘ-, and R₄ is -(CH₂)ₙ-OH, -C(=O)-Rₐ or -OH, wherein Rₐ is hydrogen, or substituted or unsubstituted C₁-C₃ alkyl, and R₅ is -OH, hydrogen, or substituted or unsubstituted C₁-C₁₅ alkyl, wherein m is an integer of 2 to 4 and n is an integer of 1 to 3, but is not limited thereto.

Also, in the compound represented by Formula 3, L₂ is - C=O-, R₄ is substituted or unsubstituted C₁-C₃ alkyl, or -O⁻Na⁺, and R₅ is hydrogen or -OH, but is not limited thereto.

Also, in the compound represented by Formula 3, L₂ is - O-, R₄ is -(CH₂)ₙ-OH, n is an integer of 1 to 3, and R₅ is hydrogen, but is not limited thereto.

In the present invention, the compound represented by Formula 3 includes at least one selected from the group consisting of cinnamaldehyde, 2-methoxycinnamaldehyde, raspberry ketone, 3-phenyl-1-propanol, 4-hydroxy acetophenone, sodium benzoate, and phenoxy EtOH, but is not limited thereto.

Specifically, the cinnamaldehyde has a structure of 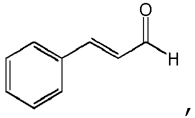 the 2-methoxycinnamaldehyde has a structure of 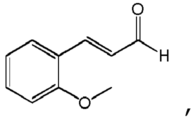 the raspberry ketone has a structure of 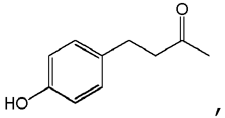 the 3-phenyl-1-propanol has a structure of 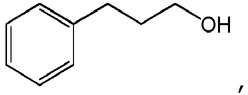 the 4-hydroxy acetophenone has a structure of 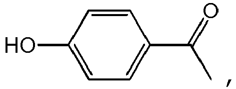 the sodium benzoate has a structure of 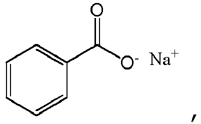 and the phenoxy EtOH has a structure of

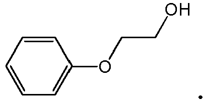

In the present invention, in the compound represented by Formula 4, L₃ is -C(=O)-, -C(CH₃)₂-CH₂-CH(CH₃)-CH₂- or - (CH₂)ₚ- , p is an integer of 5 to 12, R₆ is 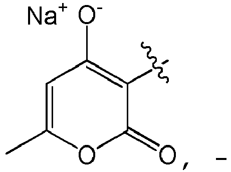 C(=O)-NH-OH, 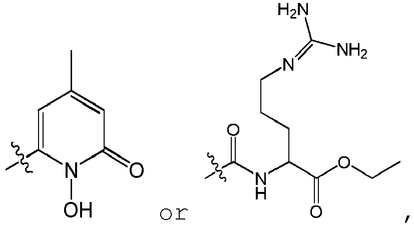 and X is NH₂-CH₂-CH₂-OH or nothing (null), but is not limited thereto.

Specifically, in the compound represented by Formula 4, L₃ is -C(=O)-, R₆ is 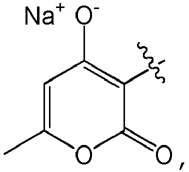 and X is nothing (null), but is not limited thereto.

Also, in the compound represented by Formula 4, L₃ is - (CH₂)ₚ-, wherein -(CH₂)ₚ- is substituted with C₁-C₃ alkyl, p is an integer of 3 to 11, R₆ is -C(=O)-NH-OH or 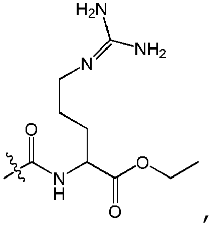 and X is nothing (null), but is not limited thereto.

Also, in the compound represented by Formula 4, L₃ is - C(CH₃) ₂-CH₂-CH(CH₃)-CH₂-, R₆ is 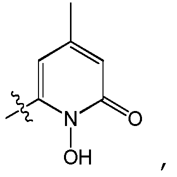 and X is NH₂-CH₂-CH₂-OH, but is not limited thereto.

In the present invention, the compound represented by Formula 4 may include at least one selected from the group consisting of sodium dehydroacetate, caprylhydroxamic acid, piroctone olamine, and lauroyl arginine ethyl ester, but is not limited thereto.

Specifically, the sodium dehydroacetate has a structure of 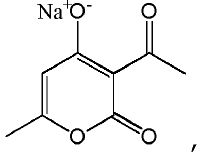 the caprylhydroxamic acid has a structure of 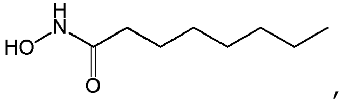 the piroctone olamine has a structure of 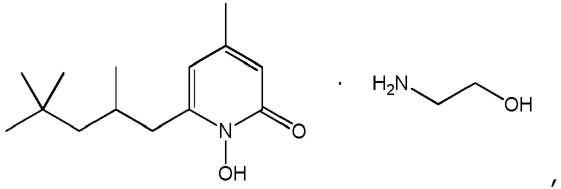 and the lauroyl arginine ethyl ester has a structure of

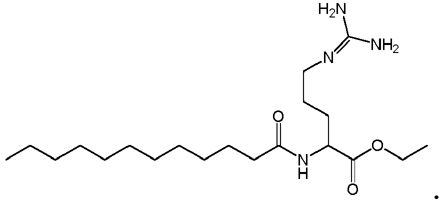

In the present invention, in the compound represented by Formula 5, R₇ is -COOH, -OR_{d} or -COO⁻Na⁺, R₈ is unsubstituted C₁-C₃ alkyl, -OH or -O⁻Na⁺, L₄ is - (CH₂)_{q}- substituted or unsubstituted with at least one substituent selected from the group consisting of hydroxy, unsubstituted C₁-C₃ alkyl and carboxyl, substituted or unsubstituted - (CH₂)ᵣ-N(Rₑ)-(CH₂)ₛ- , or unsubstituted phenylene (-C₆H₄-), R_{d} is unsubstituted C₁-C₃ alkyl, Rₑ is - (CH₂)ₜ-N[(CH₂)ᵤ-CO₂⁻)]₂, and q, r, s, t, and u are each independently an integer of 1 to 3, but is not limited thereto.

In the present invention, the compound represented by Formula 5 may be a hydrate or salt, but is not limited thereto.

In the present invention, the compound represented by Formula 5 may include at least one selected from the group consisting of levulinic acid, citric acid, sodium anisate, disodium EDTA, and tetrasodium EDTA, but is not limited thereto.

Specifically, the levulinic acid has a structure of 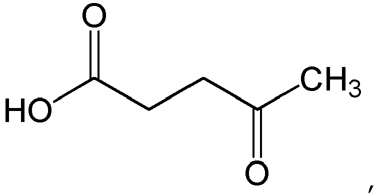 the citric acid has a structure of 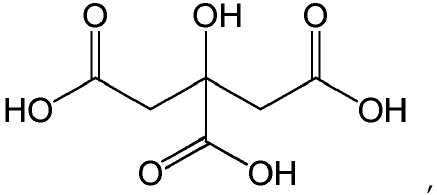 the sodium anisate has a structure of 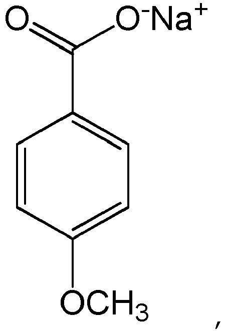 the disodium EDTA has a structure of 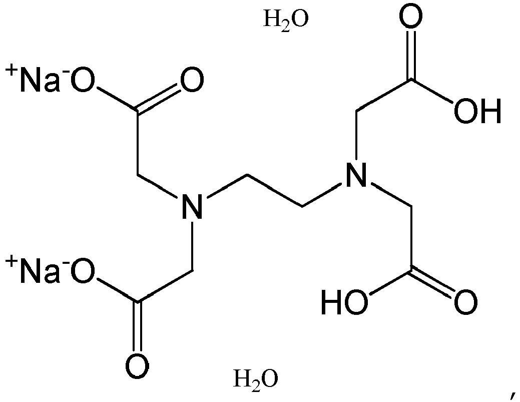 and the tetrasodium EDTA has a structure of

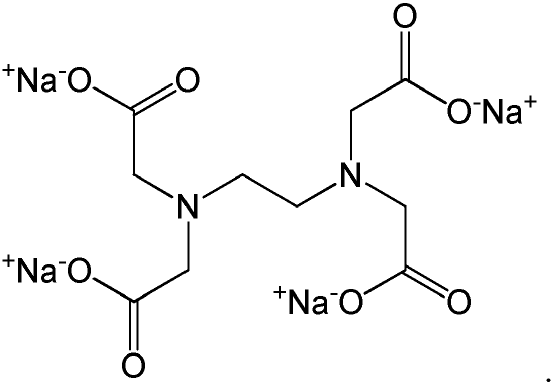

The compound represented by Formula 2, Formula 3, Formula 4 or Formula 5 is used as an ingredient of the antimicrobial and preservative composition along with a surfactant compound represented by Formula 1, thereby suppressing the growth of microorganisms such as bacteria, molds, and yeasts, preventing the growth of decaying microorganisms, and also exhibiting synergistic disinfecting activity, compared to when each compound is used alone.

### Products containing antimicrobial and preservative composition

The present invention provides a product including the antimicrobial and preservative composition.

In the present invention, the product may be a cosmetic, hygiene product or household chemical product, but is not limited thereto.

As used herein, the term "cosmetic" refers to a product that is used such as by applying to the human body, rubbing, or spraying or in a similar manner thereto, to clean and beautify the human body to add attractiveness, brighten the appearance, or maintain or promote the health of the skin and hair, while having almost no or less effects on the human body.

As used herein, the term "sanitary product" refers to a product that requires special hygiene management in order to secure health and hygiene.

As used herein, the term "household chemical product" refers to a chemical product used in everyday living spaces such as homes, offices, and multi-use facilities, which may cause exposure of chemicals to people or the environment.

In the present invention, the cosmetic may be prepared in any formulation conventionally prepared in the art according to the Cosmetics Act, and may be formulated into at least one selected from the group consisting of solutions, suspensions, emulsions, pastes, gels, creams, pacts, powders, emulsion foundations, wax foundations, sprays, soaps, face cleansers, body cleansers, hair shampoos and hair rinses, but is not limited thereto. More specifically, the cosmetic may be formulated into a softening lotion, nourishing lotion, cream, nourishing cream, massage cream, lipstick, pact, essence, eye cream, cleansing cream, cleansing foam, cleansing water, pack, or spray formulation.

In the present invention, according to the Sanitary Product Management Act, the sanitary product may be selected from the group consisting of dishwashing detergents, dishwasher detergents, and wet wipe, but is not limited thereto.

In the present invention, according to the Consumer Chemical Products and Biocides Safety Act, the household chemical product may be selected from the group consisting of laundry detergents, residential cleaners, deodorants, air cleaners, indoor air fresheners, fragrances, fabric softeners, multi-purpose cleaners, disinfectants, antimicrobials, sanitizers, and fungistats, but is not limited thereto.

In the present invention, the antimicrobial and preservative composition may be present in an amount of 0.01 to 10% by weight with respect to the weight of the cosmetic, sanitary or household chemical product, but is not limited thereto.

Specifically, the product may be selected from the group consisting of a liquid detergent composition for clothes, a dishwashing detergent composition, a stock wet wipe composition, a stock shampoo composition, a stock cosmetic cream composition, and a cosmetic pack composition, but is not limited thereto.

### Liquid detergent composition for clothes

Specifically, when the product including the antimicrobial and preservative composition is a liquid detergent composition for clothes, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of hexane-1,2-diol, octane-1,2-diol, decane-1,2-diol, hexyl glycerin, octyl glycerin, ethylhexylglycerin, and glyceryl monocaprylate, all of which are represented by Formula 2, thereby effectively controlling *E*. *coli, S. Aureus, P. Aeruginosa, A. niger* and *C*. *albicans.*

In addition, when the product including the antimicrobial and preservative composition is a liquid detergent composition for clothes, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of cinnamaldehyde, 2-methoxycinnamaldehyde, raspberry ketone, 3-phenyl-1-propanol, 4-hydroxy acetophenone, sodium benzoate, and phenoxy EtOH, all of which are represented by Formula 3, thereby effectively controlling *E*. *coli, S. Aureus, P. Aeruginosa, A. niger* and *C*. *albicans.*

In addition, when the product including the antimicrobial and preservative composition is a liquid detergent composition for clothes, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of sodium dehydroacetate, caprylhydroxamic acid, piroctone olamine, and lauroyl arginine ethyl ester, all of which are represented by Formula 4, thereby effectively controlling *E. coli, S. Aureus, P. Aeruginosa, A. niger* and *C*. *albicans.*

In addition, when the product including the antimicrobial and preservative composition is a liquid detergent composition for clothes, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of levulinic acid, citric acid, sodium anisate, disodium EDTA, and tetrasodium EDTA, all of which are represented by Formula 5, thereby effectively controlling *E*. *coli, S. Aureus, P. Aeruginosa, A. niger* and *C*. *albicans.*

### Dishwashing detergent composition

Specifically, when the product including the antimicrobial and preservative composition is a dishwashing detergent composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of hexane-1,2-diol, octane-1,2-diol, decane-1,2-diol, hexyl glycerin, octyl glycerin, ethylhexylglycerin, and glyceryl monocaprylate, all of which are represented by Formula 2, thereby effectively controlling *E. coli, S. Aureus, P. Aeruginosa, A. niger* and *C. albicans.*

In addition, when the product including the antimicrobial and preservative composition is a dishwashing detergent composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of cinnamaldehyde, 2-methoxycinnamaldehyde, raspberry ketone, 3-phenyl-1-propanol, 4-hydroxy acetophenone, sodium benzoate, and phenoxy EtOH, all of which are represented by Formula 3, thereby effectively controlling *E*. *coli, S. Aureus, P. Aeruginosa, A. niger* and C. *albicans.*

In addition, when the product including the antimicrobial and preservative composition is a dishwashing detergent composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of sodium dehydroacetate, caprylhydroxamic acid, piroctone olamine, and lauroyl arginine ethyl ester, all of which are represented by Formula 4, thereby effectively controlling *E. coli, S. Aureus, P. Aeruginosa, A. niger* and *C*. *albicans.*

In addition, when the product including the antimicrobial and preservative composition is a dishwashing detergent composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of levulinic acid, citric acid, sodium anisate, disodium EDTA, and tetrasodium EDTA, all of which are represented by Formula 5, thereby effectively controlling *E. coli, S. Aureus, P. Aeruginosa, A. niger* and *C. albicans.*

### Stock wet wipe composition

Specifically, when the product including the antimicrobial and preservative composition is a stock wet wipe composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of hexane-1,2-diol, octane-1,2-diol, decane-1,2-diol, hexyl glycerin, octyl glycerin, ethylhexylglycerin, and glyceryl monocaprylate, all of which are represented by Formula 2, thereby effectively controlling *E. coli, S. Aureus, P. Aeruginosa, A. niger* and *C. albicans.*

In addition, when the product including the antimicrobial and preservative composition is a stock wet wipe composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of cinnamaldehyde, 2-methoxycinnamaldehyde, raspberry ketone, 3-phenyl-1-propanol, 4-hydroxy acetophenone, sodium benzoate, and phenoxy EtOH, all of which are represented by Formula 3, thereby effectively controlling *E*. *coli, S. Aureus, P. Aeruginosa, A. niger* and *C*. *albicans.*

In addition, when the product including the antimicrobial and preservative composition is a stock wet wipe composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of sodium dehydroacetate, caprylhydroxamic acid, piroctone olamine, and lauroyl arginine ethyl ester, all of which are represented by Formula 4, thereby effectively controlling *E. coli, S. Aureus, P. Aeruginosa, A. niger* and *C*. *albicans.*

In addition, when the product including the antimicrobial and preservative composition is a stock wet wipe composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of levulinic acid, citric acid, sodium anisate, disodium EDTA, and tetrasodium EDTA, all of which are represented by Formula 5, thereby effectively controlling *E. coli, S. Aureus, P. Aeruginosa, A. niger* and *C. albicans.*

### Stock shampoo composition

Specifically, when the product including the antimicrobial and preservative composition is a stock shampoo composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of hexane-1,2-diol, octane-1,2-diol, decane-1,2-diol, hexyl glycerin, octyl glycerin, ethylhexylglycerin, and glyceryl monocaprylate, all of which are represented by Formula 2, thereby effectively controlling *E. coli, S. Aureus, P. Aeruginosa, A. niger* and *C. albicans.*

In addition, when the product including the antimicrobial and preservative composition is a stock shampoo composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of cinnamaldehyde, 2-methoxycinnamaldehyde, raspberry ketone, 3-phenyl-1-propanol, 4-hydroxy acetophenone, sodium benzoate, and phenoxy EtOH, all of which are represented by Formula 3, thereby effectively controlling *E*. *coli, S. Aureus, P. Aeruginosa, A. niger* and *C*. *albicans.*

In addition, when the product including the antimicrobial and preservative composition is a stock shampoo composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of sodium dehydroacetate, caprylhydroxamic acid, piroctone olamine, and lauroyl arginine ethyl ester, all of which are represented by Formula 4, thereby effectively controlling *E. coli, S. Aureus, P. Aeruginosa, A. niger* and *C*. *albicans.*

In addition, when the product including the antimicrobial and preservative composition is a stock shampoo composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of levulinic acid, citric acid, sodium anisate, disodium EDTA, and tetrasodium EDTA, all of which are represented by Formula 5, thereby effectively controlling *E. coli, S. Aureus, P. Aeruginosa, A. niger* and *C. albicans.*

### Stock cosmetic cream composition

Specifically, when the product including the antimicrobial and preservative composition is a stock cosmetic cream composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of hexane-1,2-diol, octane-1,2-diol, decane-1,2-diol, hexyl glycerin, octyl glycerin, ethylhexylglycerin, and glyceryl monocaprylate, all of which are represented by Formula 2, thereby effectively controlling *E*. *coli, S. Aureus, P. Aeruginosa, A. niger* and *C*. *albicans.*

In addition, when the product including the antimicrobial and preservative composition is a stock cosmetic cream composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of cinnamaldehyde, 2-methoxycinnamaldehyde, raspberry ketone, 3-phenyl-1-propanol, 4-hydroxy acetophenone, sodium benzoate, and phenoxy EtOH, all of which are represented by Formula 3, thereby effectively controlling *E*. *coli, S. Aureus, P. Aeruginosa, A. niger* and *C*. *albicans.*

In addition, when the product including the antimicrobial and preservative composition is a stock cosmetic cream composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of sodium dehydroacetate, caprylhydroxamic acid, piroctone olamine, and lauroyl arginine ethyl ester, all of which are represented by Formula 4, thereby effectively controlling *E. coli, S. Aureus, P. Aeruginosa, A. niger* and *C*. *albicans.*

In addition, when the product including the antimicrobial and preservative composition is a stock cosmetic cream composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of levulinic acid, citric acid, sodium anisate, disodium EDTA, and tetrasodium EDTA, all of which are represented by Formula 5, thereby effectively controlling *E*. *coli, S. Aureus, P. Aeruginosa, A. niger* and *C*. *albicans.*

### Cosmetic pact composition

Specifically, when the product including the antimicrobial and preservative composition is a cosmetic pack composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of hexane-1,2-diol, octane-1,2-diol, decane-1,2-diol, hexyl glycerin, octyl glycerin, ethylhexylglycerin, and glyceryl monocaprylate, all of which are represented by Formula 2, thereby effectively controlling *E. coli, S. Aureus, P. Aeruginosa, A. niger* and *C. albicans.*

In addition, when the product including the antimicrobial and preservative composition is a cosmetic pack composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of cinnamaldehyde, 2-methoxycinnamaldehyde, raspberry ketone, 3-phenyl-1-propanol, 4-hydroxy acetophenone, sodium benzoate, and phenoxy EtOH, all of which are represented by Formula 3, thereby effectively controlling *E*. *coli, S. Aureus, P. Aeruginosa, A. niger* and *C*. *albicans.*

In addition, when the product including the antimicrobial and preservative composition is a cosmetic pack composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of sodium dehydroacetate, caprylhydroxamic acid, piroctone olamine, and lauroyl arginine ethyl ester, all of which are represented by Formula 4, thereby effectively controlling *E. coli, S. Aureus, P. Aeruginosa, A. niger* and *C*. *albicans.*

In addition, when the product including the antimicrobial and preservative composition is a cosmetic pack composition, the product includes an antimicrobial and preservative composition containing a combination of 3-(dodecylamino)propane-1,2-diol represented by Formula 1-1 or 3-(tetradecylamino)propane-1,2-diol represented by Formula 1-2, and any one compound of levulinic acid, citric acid, sodium anisate, disodium EDTA, and tetrasodium EDTA, all of which are represented by Formula 5, thereby effectively controlling *E. coli, S. Aureus, P. Aeruginosa, A. niger* and *C. albicans.*

### Antimicrobial method

The present invention provides an antimicrobial method including treating bacteria with the antimicrobial and preservative composition.

The method for treating bacteria with the antimicrobial and preservative composition may be appropriately used according to conventional methods well known to those skilled in the art in various fields requiring antibacterial activity. The amount of the antimicrobial and preservative composition that is treated may be appropriately determined depending on the purpose of use.

Matters mentioned in the antimicrobial and preservative composition, the product including the antimicrobial and preservative composition, and the antimicrobial method of the present invention are equally applied unless contradictory to each other.

Hereinafter, various products to which the composition of the present invention is applied will be evaluated so that those skilled in the art can easily implement the present invention with reference to preferred embodiments. However, the present invention may be implemented in many different forms and is not limited to the following Example.

### Preparation Example: Preparation of antimicrobial and preservative composition

### Preparation Examples 1-7. Preparation of antimicrobial and preservative compositions using 3-(dodecylamino)propane-1,2-diol represented by Formula 1-1 and compound represented by Formula 2

### Preparation Example 1.

3-(dodecylamino)propane-1,2-diol represented by Formula 1-1 and hexane-1,2-diol represented by Formula 2 were mixed in a weight ratio of 7:3 to prepare an antimicrobial and preservative composition.

### Preparation Example 2.

3-(dodecylamino)propane-1,2-diol represented by Formula 1-1 and octane-1,2-diol represented by Formula 2 were mixed in a weight ratio of 7:3 to prepare an antimicrobial and preservative composition.

### Preparation Example 3.

3-(dodecylamino)propane-1,2-diol represented by Formula 1-1 and decane-1,2-diol represented by Formula 2 were mixed in a weight ratio of 7:3 to prepare an antimicrobial and preservative composition.

### Preparation Example 4.

3-(dodecylamino)propane-1,2-diol represented by Formula 1-1 and hexyl glycerin represented by Formula 2 were mixed in a weight ratio of 7:3 to prepare an antimicrobial and preservative composition.

### Preparation Example 5.

3-(dodecylamino)propane-1,2-diol represented by Formula 1-1 and octyl glycerin represented by Formula 2 were mixed in a weight ratio of 7:3 to prepare an antimicrobial and preservative composition.

### Preparation Example 6.

3-(dodecylamino)propane-1,2-diol represented by Formula 1-1 and ethylhexylglycerin represented by Formula 2 were mixed in a weight ratio of 7:3 to prepare an antimicrobial and preservative composition.

### Preparation Example 7.

3-(dodecylamino)propane-1,2-diol represented by Formula 1-1 and glyceryl monocaprylate represented by Formula 2 were mixed in a weight ratio of 7:3 to prepare an antimicrobial and preservative composition.

### Preparation Examples 8-14. Antimicrobial and preservative compositions using 3-(dodecylamino)propane-1,2-diol represented by Formula 1-1 and compound represented by Formula 3

### Preparation Example 8.

3-(dodecylamino)propane-1,2-diol represented by Formula 1-1 and cinnamaldehyde represented by Formula 3 were mixed in a weight ratio of 7:3 to prepare an antimicrobial and preservative composition.

### Preparation Example 9.

3-(dodecylamino)propane-1,2-diol represented by Formula 1-1 and 2-methoxycinnamaldehyde represented by Formula 3 were mixed in a weight ratio of 7:3 to prepare an antimicrobial and preservative composition.

### Preparation Example 10.

3-(dodecylamino)propane-1,2-diol represented by Formula 1-1 and raspberry ketone represented by Formula 3 were mixed in a weight ratio of 7:3 to prepare an antimicrobial and preservative composition.

### Preparation Example 11.

3-(dodecylamino)propane-1,2-diol represented by Formula 1-1 and 3-phenyl-1-propanol represented by Formula 3 were mixed in a weight ratio of 7:3 to prepare an antimicrobial and preservative composition.

### Preparation Example 12.

3-(dodecylamino)propane-1,2-diol represented by Formula 1-1 and 4-hydroxy acetophenone represented by Formula 3 were mixed in a weight ratio of 7:3 to prepare an antimicrobial and preservative composition.

### Preparation Example 13.

3-(dodecylamino)propane-1,2-diol represented by Formula 1-1 and sodium benzoate represented by Formula 3 were mixed in a weight ratio of 7:3 to prepare an antimicrobial and preservative composition.

### Preparation Example 14.

3-(dodecylamino)propane-1,2-diol represented by Formula 1-1 and phenoxy EtOH represented by Formula 3 were mixed in a weight ratio of 7:3 to prepare an antimicrobial and preservative composition.

### Preparation Examples 15-18. Antimicrobial and preservative compositions using 3-(dodecylamino)propane-1,2-diol represented by Formula 1-1 and compound represented by Formula 4

### Preparation Example 15.

3-(dodecylamino)propane-1,2-diol represented by Formula 1-1 and sodium dehydroacetate represented by Formula 4 were mixed in a weight ratio of 7:3 to prepare an antimicrobial and preservative composition.

### Preparation Example 16.

3-(dodecylamino)propane-1,2-diol represented by Formula 1-1 and caprylhydroxamic acid represented by Formula 4 were mixed in a weight ratio of 7:3 to prepare an antimicrobial and preservative composition.

### Preparation Example 17.

3-(dodecylamino)propane-1,2-diol represented by Formula 1-1 and piroctone olamine represented by Formula 4 were mixed in a weight ratio of 7:3 to prepare an antimicrobial and preservative composition.

### Preparation Example 18.

3-(dodecylamino)propane-1,2-diol represented by Formula 1-1 and lauroyl arginine ethyl ester represented by Formula 4 were mixed in a weight ratio of 7:3 to prepare an antimicrobial and preservative composition.

### Preparation Examples 19-23. Antimicrobial and preservative compositions using 3-(dodecylamino)propane-1,2-diol represented by Formula 1-1 and compound represented by Formula 5

### Preparation Example 19.

3-(dodecylamino)propane-1,2-diol represented by Formula 1-1 and levulinic acid represented by Formula 5 were mixed in a weight ratio of 7:3 to prepare an antimicrobial and preservative composition.

### Preparation Example 20.

3-(dodecylamino)propane-1,2-diol represented by Formula 1-1 and citric acid represented by Formula 5 were mixed in a weight ratio of 7:3 to prepare an antimicrobial and preservative composition.

### Preparation Example 21.

3-(dodecylamino)propane-1,2-diol represented by Formula 1-1 and sodium anisate represented by Formula 5 were mixed in a weight ratio of 7:3 to prepare an antimicrobial and preservative composition.

### Preparation Example 22.

3-(dodecylamino)propane-1,2-diol represented by Formula 1-1 and disodium EDTA represented by Formula 5 were mixed in a weight ratio of 7:3 to prepare an antimicrobial and preservative composition.

### Preparation Example 23.

3-(dodecylamino)propane-1,2-diol represented by Formula 1-1 and tetrasodium EDTA represented by Formula 5 were mixed in a weight ratio of 7:3 to prepare an antimicrobial and preservative composition.

### Comparative Examples 1~8

### Comparative Example 1.

(3-dodecylamino)propane-1,2-diol represented by Formula 1 was used.

### Comparative Example 2.

Hexane-1,2-diol represented by Formula 2 was used.

### Comparative Example 3.

Octane-1,2-diol represented by Formula 2 was used.

### Comparative Example 4.

Decane-1,2-diol represented by Formula 2 was used.

### Comparative Example 5.

Hexyl glycerin represented by Formula 2 was used.

### Comparative Example 6.

Octyl glycerin represented by Formula 2 was used.

### Comparative Example 7.

Ethylhexylglycerin represented by Formula 2 was used.

### Comparative Example 8.

Glyceryl monocaprylate represented by Formula 2 was used.

### Comparative Examples 9-15

### Comparative Example 9.

Cinnamaldehyde represented by Formula 3 was used.

### Comparative Example 10.

2-methoxycinnamaldehyde represented by Formula 3 was used.

### Comparative Example 11.

Raspberry ketone represented by Formula 3 was used.

### Comparative Example 12.

3-phenyl-1-propanol represented by Formula 3 was used.

### Comparative Example 13.

4-hydroxy acetophenone represented by Formula 3 was used.

### Comparative Example 14.

Sodium benzoate represented by Formula 3 was used.

### Comparative Example 15.

Phenoxy EtOH represented by Formula 3 was used.

### Comparative Examples 16~19

### Comparative Example 16.

Sodium dehydroacetate represented by Formula 4 was used.

### Comparative Example 17.

Caprylhydroxamic acid represented by Formula 4 was used.

### Comparative Example 18.

Piroctone olamine represented by Formula 4 was used.

### Comparative Example 19.

Lauroyl arginine ethyl ester represented by Formula 4 was used.

### Comparative Examples 20-24

### Comparative Example 20.

Levulinic acid represented by Formula 5 was used.

### Comparative Example 21.

Citric acid represented by Formula 5 was used.

### Comparative Example 22.

Sodium anisate represented by Formula 5 was used.

### Comparative Example 23.

Disodium EDTA represented by Formula 5 was used.

### Comparative Example 24.

Tetrasodium EDTA represented by Formula 5 was used.

### Example

Compounds used in the following examples were acquired from Sigma-Aldrich Korea and TCI.

### Examples 1 to 23: Liquid detergent composition for clothes

### Example 1.

A liquid detergent composition for clothes containing 6% by weight of Laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Preparation Example 1 was prepared.

### Example 2.

A liquid detergent composition for clothes was prepared in the same manner as in Example 1, except that the antimicrobial and preservative composition prepared in accordance with Preparation Example 2 was used instead of the antimicrobial and preservative composition prepared in accordance with Preparation Example 1.

### Example 3.

A liquid detergent composition for clothes was prepared in the same manner as in Example 1, except that the antimicrobial and preservative composition prepared in accordance with Preparation Example 3 was used instead of the antimicrobial and preservative composition prepared in accordance with Preparation Example 1.

### Example 4.

A liquid detergent composition for clothes was prepared in the same manner as in Example 1, except that the antimicrobial and preservative composition prepared in accordance with Preparation Example 4 was used instead of the antimicrobial and preservative composition prepared in accordance with Preparation Example 1.

### Example 5.

A liquid detergent composition for clothes was prepared in the same manner as in Example 1, except that the antimicrobial and preservative composition prepared in accordance with Preparation Example 5 was used instead of the antimicrobial and preservative composition prepared in accordance with Preparation Example 1.

### Example 6.

A liquid detergent composition for clothes was prepared in the same manner as in Example 1, except that the antimicrobial and preservative composition prepared in accordance with Preparation Example 6 was used instead of the antimicrobial and preservative composition prepared in accordance with Preparation Example 1.

### Example 7.

A liquid detergent composition for clothes was prepared in the same manner as in Example 1, except that the antimicrobial and preservative composition prepared in accordance with Preparation Example 7 was used instead of the antimicrobial and preservative composition prepared in accordance with Preparation Example 1.

### Example 8.

A liquid detergent composition for clothes containing 6% by weight of Laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Preparation Example 8 was prepared.

### Example 9.

A liquid detergent composition for clothes was prepared in the same manner as in Example 8, except that the antimicrobial and preservative composition prepared in accordance with Preparation Example 9 was used instead of the antimicrobial and preservative composition prepared in accordance with Preparation Example 8.

### Example 10.

A liquid detergent composition for clothes was prepared in the same manner as in Example 8, except that the antimicrobial and preservative composition prepared in accordance with Preparation Example 10 was used instead of the antimicrobial and preservative composition prepared in accordance with Preparation Example 8.

### Example 11.

A liquid detergent composition for clothes was prepared in the same manner as in Example 8, except that the antimicrobial and preservative composition prepared in accordance with Preparation Example 11 was used instead of the antimicrobial and preservative composition prepared in accordance with Preparation Example 8.

### Example 12.

A liquid detergent composition for clothes was prepared in the same manner as in Example 8, except that the antimicrobial and preservative composition prepared in accordance with Preparation Example 12 was used instead of the antimicrobial and preservative composition prepared in accordance with Preparation Example 8.

### Example 13.

A liquid detergent composition for clothes was prepared in the same manner as in Example 8, except that the antimicrobial and preservative composition prepared in accordance with Preparation Example 13 was used instead of the antimicrobial and preservative composition prepared in accordance with Preparation Example 8.

### Example 14.

A liquid detergent composition for clothes was prepared in the same manner as in Example 8, except that the antimicrobial and preservative composition prepared in accordance with Preparation Example 14 was used instead of the antimicrobial and preservative composition prepared in accordance with Preparation Example 8.

### Example 15.

A liquid detergent composition for clothes containing 6% by weight of Laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Preparation Example 15 was prepared.

### Example 16.

A liquid detergent composition for clothes was prepared in the same manner as in Example 15, except that the antimicrobial and preservative composition prepared in accordance with Preparation Example 16 was used instead of the antimicrobial and preservative composition prepared in accordance with Preparation Example 15.

### Example 17.

A liquid detergent composition for clothes was prepared in the same manner as in Example 15, except that the antimicrobial and preservative composition prepared in accordance with Preparation Example 17 was used instead of the antimicrobial and preservative composition prepared in accordance with Preparation Example 15.

### Example 18.

A liquid detergent composition for clothes was prepared in the same manner as in Example 15, except that the antimicrobial and preservative composition prepared in accordance with Preparation Example 18 was used instead of the antimicrobial and preservative composition prepared in accordance with Preparation Example 15.

### Example 19.

A liquid detergent composition for clothes containing 6% by weight of Laureth-7, 3% by weight of alkylbenzene sulfonate, 5% by weight of sodium laureth-2 sulfate, 0.3% by weight of protease, 0.3% by weight of lipase, 1% by weight of a coco fatty acid soap, 2% by weight of a salt, 0.2% by weight of a flavoring agent, 81.7% by weight of purified water and 0.5% by weight of the antimicrobial and preservative composition prepared in accordance with Preparation Example 19 was prepared.

### Example 20.

A liquid detergent composition for clothes was prepared in the same manner as in Example 19, except that the antimicrobial and preservative composition prepared in accordance with Preparation Example 20 was used instead of the antimicrobial and preservative composition prepared in accordance with Preparation Example 19.

### Example 21.

A liquid detergent composition for clothes was prepared in the same manner as in Example 19, except that the antimicrobial and preservative composition prepared in accordance with Preparation Example 21 was used instead of the antimicrobial and preservative composition prepared in accordance with Preparation Example 19.

### Example 22.

A liquid detergent composition for clothes was prepared in the same manner as in Example 19, except that the antimicrobial and preservative composition prepared in accordance with Preparation Example 22 was used instead of the antimicrobial and preservative composition prepared in accordance with Preparation Example 19.

### Example 23.

A liquid detergent composition for clothes was prepared in the same manner as in Example 19, except that the antimicrobial and preservative composition prepared in accordance with Preparation Example 23 was used instead of the antimicrobial and preservative composition prepared in accordance with Preparation Example 19.

### Examples 24 to 46: Dishwashing detergent composition

### Example 24.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Preparation Example 1 was prepared.

### Example 25.

A dishwashing detergent composition was prepared in the same manner as in Example 24, except that the antimicrobial and preservative composition prepared in accordance with Preparation Example 2 was used instead of the antimicrobial and preservative composition prepared in accordance with Preparation Example 1.

### Example 26.

A dishwashing detergent composition was prepared in the same manner as in Example 24, except that the antimicrobial and preservative composition prepared in accordance with Preparation Example 3 was used instead of the antimicrobial and preservative composition prepared in accordance with Preparation Example 1.

### Example 27.

A dishwashing detergent composition was prepared in the same manner as in Example 24, except that the antimicrobial and preservative composition prepared in accordance with Preparation Example 4 was used instead of the antimicrobial and preservative composition prepared in accordance with Preparation Example 1.

### Example 28.

A dishwashing detergent composition was prepared in the same manner as in Example 24, except that the antimicrobial and preservative composition prepared in accordance with Preparation Example 5 was used instead of the antimicrobial and preservative composition prepared in accordance with Preparation Example 1.

### Example 29.

A dishwashing detergent composition was prepared in the same manner as in Example 24, except that the antimicrobial and preservative composition prepared in accordance with Preparation Example 6 was used instead of the antimicrobial and preservative composition prepared in accordance with Preparation Example 1.

### Example 30.

A dishwashing detergent composition was prepared in the same manner as in Example 24, except that the antimicrobial and preservative composition prepared in accordance with Preparation Example 7 was used instead of the antimicrobial and preservative composition prepared in accordance with Preparation Example 1.

### Example 31.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Preparation Example 8 was prepared.

### Example 32.

A dishwashing detergent composition was prepared in the same manner as in Example 31, except that the antimicrobial and preservative composition prepared in accordance with Preparation Example 9 was used instead of the antimicrobial and preservative composition prepared in accordance with Preparation Example 8.

### Example 33.

A dishwashing detergent composition was prepared in the same manner as in Example 31, except that the antimicrobial and preservative composition prepared in accordance with Preparation Example 10 was used instead of the antimicrobial and preservative composition prepared in accordance with Preparation Example 8.

### Example 34.

A dishwashing detergent composition was prepared in the same manner as in Example 31, except that the antimicrobial and preservative composition prepared in accordance with Preparation Example 11 was used instead of the antimicrobial and preservative composition prepared in accordance with Preparation Example 8.

### Example 35.

A dishwashing detergent composition was prepared in the same manner as in Example 31, except that the antimicrobial and preservative composition prepared in accordance with Preparation Example 12 was used instead of the antimicrobial and preservative composition prepared in accordance with Preparation Example 8.

### Example 36.

A dishwashing detergent composition was prepared in the same manner as in Example 31, except that the antimicrobial and preservative composition prepared in accordance with Preparation Example 13 was used instead of the antimicrobial and preservative composition prepared in accordance with Preparation Example 8.

### Example 37.

A dishwashing detergent composition was prepared in the same manner as in Example 31, except that the antimicrobial and preservative composition prepared in accordance with Preparation Example 14 was used instead of the antimicrobial and preservative composition prepared in accordance with Preparation Example 8.

### Example 38.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Preparation Example 15 was prepared.

### Example 39.

A dishwashing detergent composition was prepared in the same manner as in Example 38, except that the antimicrobial and preservative composition prepared in accordance with Preparation Example 16 was used instead of the antimicrobial and preservative composition prepared in accordance with Preparation Example 15.

### Example 40.

A dishwashing detergent composition was prepared in the same manner as in Example 38, except that the antimicrobial and preservative composition prepared in accordance with Preparation Example 17 was used instead of the antimicrobial and preservative composition prepared in accordance with Preparation Example 15.

### Example 41.

A dishwashing detergent composition was prepared in the same manner as in Example 38, except that the antimicrobial and preservative composition prepared in accordance with Preparation Example 18 was used instead of the antimicrobial and preservative composition prepared in accordance with Preparation Example 15.

### Example 42.

A dishwashing detergent composition containing 5% by weight of sodium laureth-2 sulfate, 4% by weight of alpha olefin sulfate, 2% by weight of amine oxide, 2% by weight of alkyl polyglucoside, 2% by weight of alkylbenzene sulfonate, 2% by weight of glycerin, 0.2% by weight of a flavoring agent, 82.5% by weight of purified water and 0.3% by weight of the antimicrobial and preservative composition prepared in accordance with Preparation Example 19 was prepared.

### Example 43.

A dishwashing detergent composition was prepared in the same manner as in Example 42, except that the antimicrobial and preservative composition prepared in accordance with Preparation Example 20 was used instead of the antimicrobial and preservative composition prepared in accordance with Preparation Example 19.

### Example 44.

A dishwashing detergent composition was prepared in the same manner as in Example 42, except that the antimicrobial and preservative composition prepared in accordance with Preparation Example 21 was used instead of the antimicrobial and preservative composition prepared in accordance with Preparation Example 19.

### Example 45.

A dishwashing detergent composition was prepared in the same manner as in Example 42, except that the antimicrobial and preservative composition prepared in accordance with Preparation Example 22 was used instead of the antimicrobial and preservative composition prepared in accordance with Preparation Example 19.

### Example 46.

A dishwashing detergent composition was prepared in the same manner as in Example 42, except that the antimicrobial and preservative composition prepared in accordance with Preparation Example 23 was used instead of the antimicrobial and preservative composition prepared in accordance with Preparation Example 19.

### Examples 47 to 69: Stock wet wipe compositions

### Example 47.

A stock wet wipe composition containing 1% by weight of a natural extract, 98.7% by weight of purified water, and 0.3% by weight of the antimicrobial and preservative composition prepared in accordance with Preparation Example 1 was prepared.

### Example 48.

A stock wet wipe composition was prepared in the same manner as in Example 47, except that the antimicrobial and preservative composition prepared in accordance with Preparation Example 2 was used instead of the antimicrobial and preservative composition prepared in accordance with Preparation Example 1.

### Example 49.

A stock wet wipe composition was prepared in the same manner as in Example 47, except that the antimicrobial and preservative composition prepared in accordance with Preparation Example 2 was used instead of the antimicrobial and preservative composition prepared in accordance with Preparation Example 1.

### Example 50.

A stock wet wipe composition was prepared in the same manner as in Example 47, except that the antimicrobial and preservative composition prepared in accordance with Preparation Example 4 was used instead of the antimicrobial and preservative composition prepared in accordance with Preparation Example 1.

### Example 51.

A stock wet wipe composition was prepared in the same manner as in Example 47, except that the antimicrobial and preservative composition prepared in accordance with Preparation Example 5 was used instead of the antimicrobial and preservative composition prepared in accordance with Preparation Example 1.

### Example 52.

A stock wet wipe composition was prepared in the same manner as in Example 47, except that the antimicrobial and preservative composition prepared in accordance with Preparation Example 6 was used instead of the antimicrobial and preservative composition prepared in accordance with Preparation Example 1.

### Example 53.

A stock wet wipe composition was prepared in the same manner as in Example 47, except that the antimicrobial and preservative composition prepared in accordance with Preparation Example 7 was used instead of the antimicrobial and preservative composition prepared in accordance with Preparation Example 1.

### Example 54.

A stock wet wipe composition containing 1% by weight of a natural extract, 98.7% by weight of purified water, and 0.3% by weight of the antimicrobial and preservative composition prepared in accordance with Preparation Example 8 was prepared.

### Example 55.

A stock wet wipe composition was prepared in the same manner as in Example 54, except that the antimicrobial and preservative composition prepared in accordance with Preparation Example 9 was used instead of the antimicrobial and preservative composition prepared in accordance with Preparation Example 8.

### Example 56.

A stock wet wipe composition was prepared in the same manner as in Example 54, except that the antimicrobial and preservative composition prepared in accordance with Preparation Example 10 was used instead of the antimicrobial and preservative composition prepared in accordance with Preparation Example 8.

### Example 57.

A stock wet wipe composition was prepared in the same manner as in Example 54, except that the antimicrobial and preservative composition prepared in accordance with Preparation Example 11 was used instead of the antimicrobial and preservative composition prepared in accordance with Preparation Example 8.

### Example 58.

A stock wet wipe composition was prepared in the same manner as in Example 54, except that the antimicrobial and preservative composition prepared in accordance with Preparation Example 12 was used instead of the antimicrobial and preservative composition prepared in accordance with Preparation Example 8.

### Example 59.

A stock wet wipe composition was prepared in the same manner as in Example 54, except that the antimicrobial and preservative composition prepared in accordance with Preparation Example 13 was used instead of the antimicrobial and preservative composition prepared in accordance with Preparation Example 8.

### Example 60.

A stock wet wipe composition was prepared in the same manner as in Example 54, except that the antimicrobial and preservative composition prepared in accordance with Preparation Example 14 was used instead of the antimicrobial and preservative composition prepared in accordance with Preparation Example 8.

### Example 61.

A stock wet wipe composition containing 1% by weight of a natural extract, 98.7% by weight of purified water, and 0.3% by weight of the antimicrobial and preservative composition prepared in accordance with Preparation Example 15 was prepared.

### Example 62.

A stock wet wipe composition was prepared in the same manner as in Example 61, except that the antimicrobial and preservative composition prepared in accordance with Preparation Example 16 was used instead of the antimicrobial and preservative composition prepared in accordance with Preparation Example 15.

### Example 63.

A stock wet wipe composition was prepared in the same manner as in Example 61, except that the antimicrobial and preservative composition prepared in accordance with Preparation Example 17 was used instead of the antimicrobial and preservative composition prepared in accordance with Preparation Example 15.

### Example 64.

A stock wet wipe composition was prepared in the same manner as in Example 61, except that the antimicrobial and preservative composition prepared in accordance with Preparation Example 18 was used instead of the antimicrobial and preservative composition prepared in accordance with Preparation Example 15.

### Example 65.

A stock wet wipe composition containing 1% by weight of a natural extract, 98.7% by weight of purified water, and 0.3% by weight of the antimicrobial and preservative composition prepared in accordance with Preparation Example 19 was prepared.

### Example 66.

A stock wet wipe composition was prepared in the same manner as in Example 65, except that the antimicrobial and preservative composition prepared in accordance with Preparation Example 20 was used instead of the antimicrobial and preservative composition prepared in accordance with Preparation Example 19.

### Example 67.

A stock wet wipe composition was prepared in the same manner as in Example 65, except that the antimicrobial and preservative composition prepared in accordance with Preparation Example 21 was used instead of the antimicrobial and preservative composition prepared in accordance with Preparation Example 19.

### Example 68.

A stock wet wipe composition was prepared in the same manner as in Example 65, except that the antimicrobial and preservative composition prepared in accordance with Preparation Example 22 was used instead of the antimicrobial and preservative composition prepared in accordance with Preparation Example 19.

### Example 69.

A stock wet wipe composition was prepared in the same manner as in Example 65, except that the antimicrobial and preservative composition prepared in accordance with Preparation Example 23 was used instead of the antimicrobial and preservative composition prepared in accordance with Preparation Example 19.

### Examples 70 to 92: Stock shampoo compositions

### Example 70.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Preparation Example 1 were prepared.

### Example 71.

A stock shampoo composition was prepared in the same manner as in Example 70, except that the antimicrobial and preservative composition prepared in accordance with Preparation Example 2 was used instead of the antimicrobial and preservative composition prepared in accordance with Preparation Example 1.

### Example 72.

A stock shampoo composition was prepared in the same manner as in Example 70, except that the antimicrobial and preservative composition prepared in accordance with Preparation Example 3 was used instead of the antimicrobial and preservative composition prepared in accordance with Preparation Example 1.

### Example 73.

A stock shampoo composition was prepared in the same manner as in Example 70, except that the antimicrobial and preservative composition prepared in accordance with Preparation Example 4 was used instead of the antimicrobial and preservative composition prepared in accordance with Preparation Example 1.

### Example 74.

A stock shampoo composition was prepared in the same manner as in Example 70, except that the antimicrobial and preservative composition prepared in accordance with Preparation Example 5 was used instead of the antimicrobial and preservative composition prepared in accordance with Preparation Example 1.

### Example 75.

A stock shampoo composition was prepared in the same manner as in Example 70, except that the antimicrobial and preservative composition prepared in accordance with Preparation Example 6 was used instead of the antimicrobial and preservative composition prepared in accordance with Preparation Example 1.

### Example 76.

A stock shampoo composition was prepared in the same manner as in Example 70, except that the antimicrobial and preservative composition prepared in accordance with Preparation Example 7 was used instead of the antimicrobial and preservative composition prepared in accordance with Preparation Example 1.

### Example 77.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Preparation Example 8 were prepared.

### Example 78.

A stock shampoo composition was prepared in the same manner as in Example 77, except that the antimicrobial and preservative composition prepared in accordance with Preparation Example 9 was used instead of the antimicrobial and preservative composition prepared in accordance with Preparation Example 8.

### Example 79.

A stock shampoo composition was prepared in the same manner as in Example 77, except that the antimicrobial and preservative composition prepared in accordance with Preparation Example 10 was used instead of the antimicrobial and preservative composition prepared in accordance with Preparation Example 8.

### Example 80.

A stock shampoo composition was prepared in the same manner as in Example 77, except that the antimicrobial and preservative composition prepared in accordance with Preparation Example 11 was used instead of the antimicrobial and preservative composition prepared in accordance with Preparation Example 8.

### Example 81.

A stock shampoo composition was prepared in the same manner as in Example 77, except that the antimicrobial and preservative composition prepared in accordance with Preparation Example 12 was used instead of the antimicrobial and preservative composition prepared in accordance with Preparation Example 8.

### Example 82.

A stock shampoo composition was prepared in the same manner as in Example 77, except that the antimicrobial and preservative composition prepared in accordance with Preparation Example 13 was used instead of the antimicrobial and preservative composition prepared in accordance with Preparation Example 8.

### Example 83.

A stock shampoo composition was prepared in the same manner as in Example 77, except that the antimicrobial and preservative composition prepared in accordance with Preparation Example 14 was used instead of the antimicrobial and preservative composition prepared in accordance with Preparation Example 8.

### Example 84.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Preparation Example 15 were prepared.

### Example 85.

A stock shampoo composition was prepared in the same manner as in Example 84, except that the antimicrobial and preservative composition prepared in accordance with Preparation Example 16 was used instead of the antimicrobial and preservative composition prepared in accordance with Preparation Example 15.

### Example 86.

A stock shampoo composition was prepared in the same manner as in Example 84, except that the antimicrobial and preservative composition prepared in accordance with Preparation Example 17 was used instead of the antimicrobial and preservative composition prepared in accordance with Preparation Example 15.

### Example 87.

A stock shampoo composition was prepared in the same manner as in Example 84, except that the antimicrobial and preservative composition prepared in accordance with Preparation Example 18 was used instead of the antimicrobial and preservative composition prepared in accordance with Preparation Example 15.

### Example 88.

A stock shampoo composition containing 0.05% by weight of disodium EDTA, 0.1% by weight of panthenol, 1% by weight of glycerin, 1% by weight of tocopheryl acetate, 0.5% by weight of polyquaternium-7, 8% by weight of sodium laureth sulfate, 5% by weight of cocamidopropyl betaine, 2% by weight of sodium cocoyl sarcosinate, 0.5% by weight of polyquaternium-10, 1% by weight of a flavoring agent, 80.55% by weight of purified water, and 0.3% by weight of the antimicrobial and preservative composition prepared in accordance with Preparation Example 19 were prepared.

### Example 89.

A stock shampoo composition was prepared in the same manner as in Example 88, except that the antimicrobial and preservative composition prepared in accordance with Preparation Example 20 was used instead of the antimicrobial and preservative composition prepared in accordance with Preparation Example 19.

### Example 90.

A stock shampoo composition was prepared in the same manner as in Example 88, except that the antimicrobial and preservative composition prepared in accordance with Preparation Example 21 was used instead of the antimicrobial and preservative composition prepared in accordance with Preparation Example 19.

### Example 91.

A stock shampoo composition was prepared in the same manner as in Example 88, except that the antimicrobial and preservative composition prepared in accordance with Preparation Example 22 was used instead of the antimicrobial and preservative composition prepared in accordance with Preparation Example 19.

### Example 92.

A stock shampoo composition was prepared in the same manner as in Example 88, except that the antimicrobial and preservative composition prepared in accordance with Preparation Example 23 was used instead of the antimicrobial and preservative composition prepared in accordance with Preparation Example 19.

### Examples 93 to 115: Stock cosmetic cream compositions

### Example 93.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Preparation Example 1 was prepared.

### Example 94.

A stock cosmetic cream composition was prepared in the same manner as in Example 93, except that the antimicrobial and preservative composition prepared in accordance with Preparation Example 2 was used instead of the antimicrobial and preservative composition prepared in accordance with Preparation Example 1.

### Example 95.

A stock cosmetic cream composition was prepared in the same manner as in Example 93, except that the antimicrobial and preservative composition prepared in accordance with Preparation Example 3 was used instead of the antimicrobial and preservative composition prepared in accordance with Preparation Example 1.

### Example 96.

A stock cosmetic cream composition was prepared in the same manner as in Example 93, except that the antimicrobial and preservative composition prepared in accordance with Preparation Example 4 was used instead of the antimicrobial and preservative composition prepared in accordance with Preparation Example 1.

### Example 97.

A stock cosmetic cream composition was prepared in the same manner as in Example 93, except that the antimicrobial and preservative composition prepared in accordance with Preparation Example 5 was used instead of the antimicrobial and preservative composition prepared in accordance with Preparation Example 1.

### Example 98.

A stock cosmetic cream composition was prepared in the same manner as in Example 93, except that the antimicrobial and preservative composition prepared in accordance with Preparation Example 6 was used instead of the antimicrobial and preservative composition prepared in accordance with Preparation Example 1.

### Example 99.

A stock cosmetic cream composition was prepared in the same manner as in Example 93, except that the antimicrobial and preservative composition prepared in accordance with Preparation Example 7 was used instead of the antimicrobial and preservative composition prepared in accordance with Preparation Example 1.

### Example 100.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Preparation Example 8 was prepared.

### Example 101.

A stock cosmetic cream composition was prepared in the same manner as in Example 100, except that the antimicrobial and preservative composition prepared in accordance with Preparation Example 9 was used instead of the antimicrobial and preservative composition prepared in accordance with Preparation Example 8.

### Example 102.

A stock cosmetic cream composition was prepared in the same manner as in Example 100, except that the antimicrobial and preservative composition prepared in accordance with Preparation Example 10 was used instead of the antimicrobial and preservative composition prepared in accordance with Preparation Example 8.

### Example 103.

A stock cosmetic cream composition was prepared in the same manner as in Example 100, except that the antimicrobial and preservative composition prepared in accordance with Preparation Example 11 was used instead of the antimicrobial and preservative composition prepared in accordance with Preparation Example 8.

### Example 104.

A stock cosmetic cream composition was prepared in the same manner as in Example 100, except that the antimicrobial and preservative composition prepared in accordance with Preparation Example 12 was used instead of the antimicrobial and preservative composition prepared in accordance with Preparation Example 8.

### Example 105.

A stock cosmetic cream composition was prepared in the same manner as in Example 100, except that the antimicrobial and preservative composition prepared in accordance with Preparation Example 13 was used instead of the antimicrobial and preservative composition prepared in accordance with Preparation Example 8.

### Example 106.

A stock cosmetic cream composition was prepared in the same manner as in Example 100, except that the antimicrobial and preservative composition prepared in accordance with Preparation Example 14 was used instead of the antimicrobial and preservative composition prepared in accordance with Preparation Example 8.

### Example 107.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Preparation Example 15 was prepared.

### Example 108.

A stock cosmetic cream composition was prepared in the same manner as in Example 107, except that the antimicrobial and preservative composition prepared in accordance with Preparation Example 16 was used instead of the antimicrobial and preservative composition prepared in accordance with Preparation Example 15.

### Example 109.

A stock cosmetic cream composition was prepared in the same manner as in Example 107, except that the antimicrobial and preservative composition prepared in accordance with Preparation Example 17 was used instead of the antimicrobial and preservative composition prepared in accordance with Preparation Example 15.

### Example 110.

A stock cosmetic cream composition was prepared in the same manner as in Example 107, except that the antimicrobial and preservative composition prepared in accordance with Preparation Example 18 was used instead of the antimicrobial and preservative composition prepared in accordance with Preparation Example 15.

### Example 111.

A stock cosmetic cream composition containing 2.5% by weight of cetostearyl alcohol, 1.5% by weight of glyceryl stearate, 5.0% by weight of trioctanoin, 1.2% by weight of polysorbate 60, 0.5% by weight of sorbitan stearate, 5.0% by weight of squalane, 3.0% by weight of liquid paraffin, 3.0% by weight of cyclomethicone, 0.2% by weight of delta-tocopherol, 4.0% by weight of glycerin, 2.0% by weight of 1,3-butylene glycol, 0.2% by weight of xanthan gum, 0.05% by weight of EDTA-2Na, 0.1% by weight of a flavoring agent, 71.55% by weight of purified water, and 0.2% by weight of the antimicrobial and preservative composition prepared in accordance with Preparation Example 19 was prepared.

### Example 112.

A stock cosmetic cream composition was prepared in the same manner as in Example 111, except that the antimicrobial and preservative composition prepared in accordance with Preparation Example 20 was used instead of the antimicrobial and preservative composition prepared in accordance with Preparation Example 19.

### Example 113.

A stock cosmetic cream composition was prepared in the same manner as in Example 111, except that the antimicrobial and preservative composition prepared in accordance with Preparation Example 21 was used instead of the antimicrobial and preservative composition prepared in accordance with Preparation Example 19.

### Example 114.

A stock cosmetic cream composition was prepared in the same manner as in Example 111, except that the antimicrobial and preservative composition prepared in accordance with Preparation Example 22 was used instead of the antimicrobial and preservative composition prepared in accordance with Preparation Example 19.

### Example 115.

A stock cosmetic cream composition was prepared in the same manner as in Example 111, except that the antimicrobial and preservative composition prepared in accordance with Preparation Example 23 was used instead of the antimicrobial and preservative composition prepared in accordance with Preparation Example 19.

### Examples 116 to 138: Cosmetic pact (solid color) compositions

### Example 116.

A Cosmetic pact (solid color) composition containing 25% by weight of a spherical powder (Silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Preparation Example 1 was prepared.

### Example 117.

A Cosmetic pact composition was prepared in the same manner as in Example 116, except that the antimicrobial and preservative composition prepared in accordance with Preparation Example 2 was used instead of the antimicrobial and preservative composition prepared in accordance with Preparation Example 1.

### Example 118.

A Cosmetic pact composition was prepared in the same manner as in Example 116, except that the antimicrobial and preservative composition prepared in accordance with Preparation Example 3 was used instead of the antimicrobial and preservative composition prepared in accordance with Preparation Example 1.

### Example 119.

A Cosmetic pact composition was prepared in the same manner as in Example 116, except that the antimicrobial and preservative composition prepared in accordance with Preparation Example 4 was used instead of the antimicrobial and preservative composition prepared in accordance with Preparation Example 1.

### Example 120.

A Cosmetic pact composition was prepared in the same manner as in Example 116, except that the antimicrobial and preservative composition prepared in accordance with Preparation Example 5 was used instead of the antimicrobial and preservative composition prepared in accordance with Preparation Example 1.

### Example 121.

A Cosmetic pact composition was prepared in the same manner as in Example 116, except that the antimicrobial and preservative composition prepared in accordance with Preparation Example 6 was used instead of the antimicrobial and preservative composition prepared in accordance with Preparation Example 1.

### Example 122.

A Cosmetic pact composition was prepared in the same manner as in Example 116, except that the antimicrobial and preservative composition prepared in accordance with Preparation Example 7 was used instead of the antimicrobial and preservative composition prepared in accordance with Preparation Example 1.

### Example 123.

A Cosmetic pact (solid color) composition containing 25% by weight of a spherical powder (Silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Preparation Example 8 was prepared.

### Example 124.

A Cosmetic pact composition was prepared in the same manner as in Example 123, except that the antimicrobial and preservative composition prepared in accordance with Preparation Example 9 was used instead of the antimicrobial and preservative composition prepared in accordance with Preparation Example 8.

### Example 125.

A Cosmetic pact composition was prepared in the same manner as in Example 123, except that the antimicrobial and preservative composition prepared in accordance with Preparation Example 10 was used instead of the antimicrobial and preservative composition prepared in accordance with Preparation Example 8.

### Example 126.

A Cosmetic pact composition was prepared in the same manner as in Example 123, except that the antimicrobial and preservative composition prepared in accordance with Preparation Example 11 was used instead of the antimicrobial and preservative composition prepared in accordance with Preparation Example 8.

### Example 127.

A Cosmetic pact composition was prepared in the same manner as in Example 123, except that the antimicrobial and preservative composition prepared in accordance with Preparation Example 12 was used instead of the antimicrobial and preservative composition prepared in accordance with Preparation Example 8.

### Example 128.

A Cosmetic pact composition was prepared in the same manner as in Example 123, except that the antimicrobial and preservative composition prepared in accordance with Preparation Example 13 was used instead of the antimicrobial and preservative composition prepared in accordance with Preparation Example 8.

### Example 129.

A Cosmetic pact composition was prepared in the same manner as in Example 123, except that the antimicrobial and preservative composition prepared in accordance with Preparation Example 14 was used instead of the antimicrobial and preservative composition prepared in accordance with Preparation Example 8.

### Example 130.

A Cosmetic pact (solid color) composition containing 25% by weight of a spherical powder (Silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Preparation Example 15 was prepared.

### Example 131.

A Cosmetic pact composition was prepared in the same manner as in Example 130, except that the antimicrobial and preservative composition prepared in accordance with Preparation Example 16 was used instead of the antimicrobial and preservative composition prepared in accordance with Preparation Example 15.

### Example 132.

A Cosmetic pact composition was prepared in the same manner as in Example 130, except that the antimicrobial and preservative composition prepared in accordance with Preparation Example 17 was used instead of the antimicrobial and preservative composition prepared in accordance with Preparation Example 15.

### Example 133.

A Cosmetic pact composition was prepared in the same manner as in Example 130, except that the antimicrobial and preservative composition prepared in accordance with Preparation Example 18 was used instead of the antimicrobial and preservative composition prepared in accordance with Preparation Example 15.

### Example 134.

A Cosmetic pact (solid color) composition containing 25% by weight of a spherical powder (Silica-HDI), 10% by weight of calcium aluminum borosilicate, 20% by weight of an oil binder silicone oil (dimethicone), 0.2% by weight of a flavoring agent, 5% by weight of a pigment, 20% by weight of ethanol, 19.4% by weight of a plate-shaped talc powder (Talc JA 46R) and 0.4% by weight of the antimicrobial and preservative composition prepared according to Preparation Example 19 was prepared.

### Example 135.

A Cosmetic pact composition was prepared in the same manner as in Example 134, except that the antimicrobial and preservative composition prepared in accordance with Preparation Example 20 was used instead of the antimicrobial and preservative composition prepared in accordance with Preparation Example 19.

### Example 136.

A Cosmetic pact composition was prepared in the same manner as in Example 134, except that the antimicrobial and preservative composition prepared in accordance with Preparation Example 21 was used instead of the antimicrobial and preservative composition prepared in accordance with Preparation Example 19.

### Example 137.

A Cosmetic pact composition was prepared in the same manner as in Example 134, except that the antimicrobial and preservative composition prepared in accordance with Preparation Example 22 was used instead of the antimicrobial and preservative composition prepared in accordance with Preparation Example 19.

### Example 138.

A Cosmetic pact composition was prepared in the same manner as in Example 134, except that the antimicrobial and preservative composition prepared in accordance with Preparation Example 23 was used instead of the antimicrobial and preservative composition prepared in accordance with Preparation Example 19.

### Experimental example

Antimicrobial tests against each of bacteria, fungi, and yeasts were performed on each of the antimicrobial and preservative compositions prepared in accordance with the compositions of Comparative Examples and Preparation Examples, and household chemical product compositions prepared in accordance with Examples as follows.

### [Method] Antimicrobial test

The antimicrobial activity of the test solution was measured using a broth dilution method. The concentration of the test solution that was treated was diluted from the highest concentration of 5% to the lowest concentration of 0.0001%. The test solution was cultured in the presence of the strain and then the turbidity of the tube was determined. At this time, the concentration of the test solution in the tube where no bacterial growth was observed among the tubes treated with the test solution at each concentration was determined as a minimum inhibitory concentration (MIC), compared to the degree of turbidity observed in the tube inoculated with only the strain without treatment with the test solution. After the primary test described as above, the concentration of the test solution that was treated was further subdivided and a secondary test was conducted to obtain an accurate minimum inhibitory concentration (MIC).

### 1) Pre-culture of test bacteria

Test strains *E. coli, S. aureus,* and *P. aeruginosa* were inoculated into tryptic soy broth and incubated at 35±2°C for 16 to 24 hours. *C. albicans* was inoculated into Sabouraud dextrose broth and cultured at 35°C for 24 hours. *A. niger* was seeded on potato dextrose agar and cultured at (25±1)°C for 7 days, sterile physiological saline was dispensed into a solid medium on which the bacteria grew, and spores were removed using a spreader and then diluted in sterile physiological saline.

### 2) Preparation of test solution

The antimicrobial and preservative compositions of Preparation Examples 1 to 23 were dissolved in dipropylene glycol and the resulting solutions were used as test solutions.

Comparative Examples 1 to 24 were used as comparative solutions.

### 3) Mixing of strain with antimicrobial and preservative composition (test solution)

A liquid medium was put into the tube and the concentration of the test solution that was treated was diluted from the highest concentration of 5% to the lowest concentration of 0.0001%. The liquid media used for *E. coli, S. aureus, and P. aeruginosa* (bacteria) were tryptic soy broth, and the liquid media used for *C*. *albicans,* and *A. niger* (fungi) were Sabouraud dextrose broth.

### 4) Inoculation of test bacterial solution

*E. coli, S. aureus,* and *P. aeruginosa* of the strains were inoculated at a concentration of 1×10⁵ CFU/mL in the test tube. The tubes treated with the test solution were incubated at 35±2°C for 16 to 24 hours. *C. albicans* and *A. niger* were inoculated at a concentration of 1×10⁴ CFU/mL in the test tube. After treatment with the test solution was completed, *C. albicans* was cultured at 35°C for 24 to 48 hours and *A*. *niger* was cultured at 25°C for 46 to 50 hours. A medium tube, to which the bacterial solution was not added, was used as a negative control, and a tube containing the medium inoculated with only the bacterial solution without the test solution was used as a growth control. In addition, in order to determine the antimicrobial activity of dipropylene glycol itself used as a solvent, the MIC of the test bacteria for the solvent was measured under the same conditions as in the sample.

### [Test result]

The test results are shown in [Table 1] to [Table 32], and the test results showed that the antimicrobial and preservative compositions of the present invention, and cosmetic, hygiene and household chemical product compositions including the antimicrobial and preservative composition of the present invention effectively controlled bacteria, filamentous fungi, and yeasts.

Specifically, Tables 1 to 4 show the antimicrobial effects of Comparative Examples, and Tables 5 to 8 show the results of antimicrobial tests against bacteria, filamentous fungi (molds), and yeasts of the antimicrobial and preservative compositions prepared in accordance with Preparation Examples of the present invention.

**[Table 1]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Comparati ve Example 1 | 0.01 | 0.01 | 0.03 | 0.09 | 0.01 |
| Comparati ve Example 2 | 1 | 2 | 1 | 0.5 | 2 |
| Comparati ve Example 3 | 0.25 | 0.5 | 0.5 | 0.3 | 0.5 |
| Comparati ve Example 4 | 0.09 | 0.025 | 0.045 | 0.006 | 0.011 |
| Comparati ve Example 5 | 0.2 | 0.5 | 0.5 | 0.32 | 0.64 |
| Comparati ve Example 6 | 0.5 | 0.5 | 0.5 | 0.5 | 0.25 |
| Comparati ve Example 7 | 0.3 | 0.15 | 2 | 0.5 | 0.5 |
| Comparati ve Example 8 | 0.12 | 0.12 | 0.12 | 0.5 | 0.5 |

**[Table 2]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Comparati ve Example 9 | 0.05 | 0.03 | 0.1 | 0.03 | 0.02 |
| Comparati ve Example 10 | 0.02 | 0.02 | 0.4 | 0.005 | 0.005 |
| Comparati ve Example 11 | 0.5 | 0.1 | 0.1 | 0.5 | 1 |
| Comparati ve Example 12 | 0.4 | 0.4 | 0.2 | 0.03 | 0.03 |
| Comparati ve Example 13 | 0.15 | 0.2 | 0.1 | 0.5 | 0.1 |
| Comparati ve Example 14 | 0.016 | 0.002 | 0.016 | 0.1 | 0.12 |
| Comparati ve Example 15 | 0.06 | 0.06 | 0.09 | 0.12 | 0.12 |

**[Table 3]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Comparati ve Example 16 | 0.1 | 0.1 | 0.1 | 0.2 | 0.1 |
| Comparati ve Example 17 | 0.06 | 0.06 | 0.12 | 0.02 | 0.03 |
| Comparati ve Example 18 | 0.05 | 0.05 | 0.2 | 0.3 | 0.2 |
| Comparati ve Example 19 | 0.003 | 0.003 | 0.003 | 0.003 | 0.001 |

**[Table 4]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Comparati ve Example 20 | 0.02 | 0.02 | 0.1 | 0.15 | 0.1 |
| Comparati ve Example 21 | 0.1 | 0.1 | 0.1 | 0.2 | 0.05 |
| Comparati ve Example 22 | 0.05 | 0.01 | 0.05 | 0.1 | 0.1 |
| Comparati ve Example 23 | 0.1 | 0.1 | 0.15 | 0.15 | 0.05 |
| Comparati ve Example 24 | 0.1 | 0.1 | 0.15 | 0.15 | 0.05 |

**[Table 5]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Preparati on Example 1 | 0.007 | 0.007 | 0.01 | 0.03 | 0.007 |
| Preparati on Example 2 | 0.005 | 0.005 | 0.007 | 0.01 | 0.005 |
| Preparati on Example 3 | 0.002 | 0.002 | 0.005 | 0.005 | 0.002 |
| Preparati on Example 4 | 0.007 | 0.007 | 0.01 | 0.05 | 0.007 |
| Preparati on Example 5 | 0.01 | 0.007 | 0.01 | 0.03 | 0.007 |
| Preparati on Example 6 | 0.007 | 0.007 | 0.01 | 0.03 | 0.007 |
| Preparati on Example 7 | 0.007 | 0.005 | 0.007 | 0.01 | 0.005 |

**[Table 6]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Preparati on Example 8 | 0.007 | 0.005 | 0.005 | 0.01 | 0.007 |
| Preparati on Example 9 | 0.001 | 0.001 | 0.002 | 0.002 | 0.001 |
| Preparati on Example 10 | 0.007 | 0.007 | 0.01 | 0.03 | 0.007 |
| Preparati on Example 11 | 0.01 | 0.007 | 0.005 | 0.01 | 0.005 |
| Preparati on Example 12 | 0.005 | 0.005 | 0.02 | 0.03 | 0.007 |
| Preparati on Example 13 | 0.001 | 0.001 | 0.001 | 0.005 | 0.005 |
| Preparati on Example 14 | 0.01 | 0.007 | 0.01 | 0.03 | 0.01 |

**[Table 7]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Preparati on Example 15 | 0.005 | 0.005 | 0.01 | 0.03 | 0.005 |
| Preparati on Example 16 | 0.001 | 0.001 | 0.005 | 0.002 | 0.005 |
| Preparati on Example 17 | 0.007 | 0.005 | 0.007 | 0.03 | 0.007 |
| Preparati on Example 18 | 0.001 | 0.0005 | 0.001 | 0.001 | 0.0005 |

**[Table 8]**

| (%) | Bacteria | | | Flamento us fungi (molds) | Yeasts |
|---|---|---|---|---|---|
| | *E. col i* | *S. Aureu s* | *P.Aerugino sa* | *A. niger* | *C.albica ns* |
| Preparati on Example 19 | 0.005 | 0.001 | 0.007 | 0.03 | 0.005 |
| Preparati on Example 20 | 0.001 | 0.001 | 0.005 | 0.02 | 0.003 |
| Preparati on Example 21 | 0.005 | 0.003 | 0.003 | 0.05 | 0.005 |
| Preparati on Example 22 | 0.003 | 0.001 | 0.005 | 0.05 | 0.03 |
| Preparati on Example 23 | 0.003 | 0.001 | 0.005 | 0.05 | 0.03 |

As can be seen from Tables 1 and 5, all antimicrobial and preservative compositions prepared in accordance with the present invention exhibited excellent antimicrobial effects against bacteria, filamentous fungi, and yeasts, compared to the single compound of Table 1, and in particular, the antimicrobial and preservative composition containing a combination of 3-(dodecylamino)propane-1,2-diol and decane-1,2-diol according to Preparation Example 3 effectively controlled all of bacteria (*E. coli, S. Aureus, P. Aeruginosa*), filamentous fungi (*A. niger*) and yeasts (*C. albicans*)*.*

As can be seen from Tables 1, 2 and 6, all antimicrobial and preservative compositions prepared according to the present invention exhibited excellent antimicrobial effects against bacteria, filamentous fungi, and yeasts compared to the single compounds in Tables 1 and 2, and in particular, the antimicrobial and preservative composition containing a combination of 3-(dodecylamino)propane-1,2-diol and sodium benzoate according to Preparation Example 13 effectively controlled bacteria (*E. coli, S. Aureus,* and *P. Aeruginosa*)*.*

As can be seen from Tables 1, 3 and 7, all antimicrobial and preservative compositions prepared according to the present invention exhibited excellent antimicrobial effects against bacteria, filamentous fungi and yeasts compared to the single compounds in Tables 1 and 3, and in particular, the antimicrobial and preservative composition containing a combination of 3-(dodecylamino)propane-1,2-diol and lauroyl arginine ethyl ester according to Preparation Example 18 effectively controlled all of bacteria (*E. coli, S. Aureus, P. Aeruginosa*)*,* filamentous fungi (*A. niger*)*,* and yeasts (*C*. *albicans*)*.*

As can be seen from Tables 1, 4 and 8, all antimicrobial and preservative compositions prepared according to the present invention exhibited excellent antimicrobial effects against bacteria, filamentous fungi and yeasts compared to the single compounds of Tables 1 and 4, and in particular, the antimicrobial and preservative composition containing a combination of 3-(dodecylamino)propane-1,2-diol and citric acid according to Preparation Example 20 effectively controlled all of bacteria (*E. coli, S. Aureus, P. Aeruginosa*), filamentous fungi (*A. niger*)*,* and yeasts (*C*. *albicans*)*.*

### [Tables 9 to 12] Antimicrobial test results of liquid detergent compositions for clothes

The following Table 9 shows the results of antimicrobial tests against bacteria, filamentous fungi, and yeasts of the liquid detergent compositions for clothes of Examples 1 to 7.

**[Table 9]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 1 | 0.01 | 0.05 | 0.03 | 0.05 | 0.03 |
| Exampl e 2 | 0.007 | 0.007 | 0.01 | 0.02 | 0.01 |
| Exampl e 3 | 0.005 | 0.005 | 0.01 | 0.007 | 0.005 |
| Exampl e 4 | 0.01 | 0.01 | 0.02 | 0.05 | 0.01 |
| Exampl e 5 | 0.01 | 0.01 | 0.03 | 0.05 | 0.02 |
| Exampl e 6 | 0.01 | 0.01 | 0.01 | 0.05 | 0.01 |
| Exampl e 7 | 0.007 | 0.007 | 0.01 | 0.01 | 0.01 |

As can be seen from Table 9, the liquid detergent compositions for clothes according to Examples 1 to 7 effectively controlled all of bacteria (*E. coli, S. Aureus, P. Aeruginosa*), filamentous fungi (*A*. *niger*), and yeasts (*C. albicans*) used for the antimicrobial tests.

The following Table 10 shows the results of antimicrobial tests against bacteria, filamentous fungi, and yeasts of the liquid detergent compositions for clothes of Examples 8 to 14.

**[Table 101**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 8 | 0.01 | 0.007 | 0.01 | 0.02 | 0.01 |
| Exampl e 9 | 0.005 | 0.002 | 0.005 | 0.005 | 0.005 |
| Exampl e 10 | 0.01 | 0.01 | 0.02 | 0.05 | 0.01 |
| Exampl e 11 | 0.01 | 0.01 | 0.007 | 0.01 | 0.007 |
| Exampl e 12 | 0.01 | 0.007 | 0.03 | 0.05 | 0.01 |
| Exampl e 13 | 0.005 | 0.005 | 0.005 | 0.01 | 0.007 |
| Exampl e 14 | 0.02 | 0.01 | 0.03 | 0.05 | 0.03 |

As can be seen from Table 10, the liquid detergent compositions for clothes according to Examples 8 to 14 effectively controlled all of bacteria (*E. coli, S. Aureus, P. Aeruginosa*), filamentous fungi (*A. niger),* and yeasts (*C. albicans*) used for the antimicrobial tests.

The following Table 11 shows the results of antimicrobial tests against bacteria, filamentous fungi, and yeasts of the liquid detergent compositions for clothes of Examples 15 to 18.

**[Table 11]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 15 | 0.007 | 0.007 | 0.01 | 0.03 | 0.01 |
| Exampl e 16 | 0.005 | 0.002 | 0.005 | 0.005 | 0.005 |
| Exampl e 17 | 0.01 | 0.01 | 0.01 | 0.05 | 0.01 |
| Exampl e 18 | 0.005 | 0.001 | 0.002 | 0.003 | 0.001 |

As can be seen from Table 11, the liquid detergent compositions for clothes according to Examples 15 to 18 effectively controlled all of bacteria (*E. coli, S. Aureus, P. Aeruginosa*), filamentous fungi (*A. niger*), and yeasts (*C*. *albicans*) used for the antimicrobial tests.

The following Table 12 shows the results of antimicrobial tests against bacteria, filamentous fungi, and yeasts of the liquid detergent compositions for clothes of Examples 19 to 23.

**[Table 12]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 19 | 0.007 | 0.005 | 0.01 | 0.05 | 0.007 |
| Exampl e 20 | 0.005 | 0.003 | 0.01 | 0.05 | 0.005 |
| Exampl e 21 | 0.007 | 0.005 | 0.005 | 0.08 | 0.07 |
| Exampl e 22 | 0.005 | 0.005 | 0.01 | 0.07 | 0.05 |
| Exampl e 23 | 0.005 | 0.005 | 0.01 | 0.07 | 0.05 |

As can be seen from Table 12, the liquid detergent compositions for clothes according to Examples 19 to 23 effectively controlled all of bacteria (*E. coli, S. Aureus, P. Aeruginosa*), filamentous fungi (*A. niger),* and yeasts (*C. albicans*) used for the antimicrobial tests.

### [Tables 13 to 16] Antimicrobial test results of dishwashing detergent compositions

The following Table 13 shows the results of the antimicrobial tests against bacteria, filamentous fungi, and yeasts of the dishwashing detergent compositions of Examples 24 to 30.

**[Table 13]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Example 24 | 0.03 | 0.03 | 0.03 | 0.09 | 0.02 |
| Example 25 | 0.01 | 0.007 | 0.01 | 0.02 | 0.01 |
| Example 26 | 0.005 | 0.005 | 0.007 | 0.007 | 0.003 |
| Example 27 | 0.02 | 0.01 | 0.02 | 0.05 | 0.01 |
| Example 28 | 0.01 | 0.01 | 0.02 | 0.03 | 0.01 |
| Example 29 | 0.01 | 0.01 | 0.02 | 0.03 | 0.01 |
| Example 30 | 0.007 | 0.007 | 0.01 | 0.01 | 0.007 |

As can be seen from Table 13, the dishwashing detergent compositions according to Examples 24 to 30 effectively controlled all of bacteria (*E. coli, S. Aureus, P. Aeruginosa*), filamentous fungi (*A*. *niger*), and yeasts (*C. albicans*) used for the antimicrobial tests.

The following Table 14 shows the results of antimicrobial tests against bacteria, filamentous fungi, and yeasts of the dishwashing detergent compositions of Examples 31 to 37.

**[Table 14]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 31 | 0.01 | 0.01 | 0.01 | 0.02 | 0.007 |
| Exampl e 32 | 0.002 | 0.002 | 0.005 | 0.005 | 0.002 |
| Exampl e 33 | 0.01 | 0.01 | 0.03 | 0.05 | 0.02 |
| Exampl e 34 | 0.01 | 0.007 | 0.01 | 0.02 | 0.01 |
| Exampl e 35 | 0.007 | 0.007 | 0.03 | 0.03 | 0.01 |
| Exampl e 36 | 0.002 | 0.002 | 0.005 | 0.01 | 0.01 |
| Exampl e 37 | 0.02 | 0.02 | 0.03 | 0.05 | 0.02 |

As can be seen from Table 14, the dishwashing detergent compositions according to Examples 31 to 37 effectively controlled all of bacteria (*E. coli, S. Aureus, P. Aeruginosa*), filamentous fungi (*A*. *niger*), and yeasts (*C. albicans*) used for the antimicrobial tests.

The following Table 15 shows the results of antimicrobial tests against bacteria, filamentous fungi, and yeasts of the dishwashing detergent compositions of Examples 38 to 41.

**[Table 15]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 38 | 0.01 | 0.007 | 0.02 | 0.05 | 0.01 |
| Exampl e 39 | 0.005 | 0.005 | 0.005 | 0.007 | 0.007 |
| Exampl e 40 | 0.01 | 0.01 | 0.02 | 0.05 | 0.01 |
| Exampl e 41 | 0.007 | 0.001 | 0.003 | 0.005 | 0.001 |

As can be seen from Table 15, the dishwashing detergent compositions according to Examples 38 to 41 effectively controlled all of bacteria (*E. coli, S. Aureus, P. Aeruginosa*), filamentous fungi (*A. niger),* and yeasts (*C*. *albicans*) used for the antimicrobial tests.

The following Table 16 shows the results of antimicrobial tests against bacteria, filamentous fungi, and yeasts of the dishwashing detergent compositions of Examples 42 to 46.

**[Table 16]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 42 | 0.01 | 0.007 | 0.01 | 0.05 | 0.01 |
| Exampl e 43 | 0.007 | 0.005 | 0.02 | 0.07 | 0.01 |
| Exampl e 44 | 0.007 | 0.007 | 0.01 | 0.05 | 0.05 |
| Exampl e 45 | 0.005 | 0.005 | 0.03 | 0.07 | 0.02 |
| Exampl e 46 | 0.005 | 0.005 | 0.03 | 0.07 | 0.01 |

As can be seen from Table 16, the dishwashing detergent compositions according to Examples 42 to 46 effectively controlled all of bacteria (*E. coli, S. Aureus, P. Aeruginosa*), filamentous fungi (*A. niger*), and yeasts (*C. albicans*) used for the antimicrobial tests.

### [Tables 17 to 20] Antimicrobial test results of stock wet wipe compositions

The following Table 17 shows the results of the antimicrobial tests against bacteria, filamentous fungi, and yeasts of the stock wet wipe compositions of Examples 47 to 53.

**[Table 17]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 47 | 0.03 | 0.05 | 0.03 | 0.05 | 0.05 |
| Exampl e 48 | 0.01 | 0.01 | 0.01 | 0.03 | 0.01 |
| Exampl e 49 | 0.007 | 0.005 | 0.007 | 0.01 | 0.005 |
| Exampl e 50 | 0.01 | 0.01 | 0.02 | 0.07 | 0.02 |
| Exampl e 51 | 0.01 | 0.01 | 0.01 | 0.03 | 0.01 |
| Exampl e 52 | 0.01 | 0.007 | 0.01 | 0.03 | 0.01 |
| Exampl e 53 | 0.007 | 0.007 | 0.007 | 0.01 | 0.01 |

As can be seen from Table 17, the stock wet wipe compositions according to Examples 47 to 53 effectively controlled all of bacteria (*E. coli, S. Aureus, P. Aeruginosa*), filamentous fungi (*A. niger),* and yeasts (*C. albicans*) used for the antimicrobial tests.

The following Table 18 shows the results of antimicrobial tests against bacteria, filamentous fungi, and yeasts of the stock wet wipe compositions of Examples 54 to 60.

**[Table 18]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 54 | 0.01 | 0.005 | 0.02 | 0.03 | 0.01 |
| Exampl e 55 | 0.007 | 0.002 | 0.007 | 0.007 | 0.002 |
| Exampl e 56 | 0.02 | 0.01 | 0.02 | 0.05 | 0.02 |
| Exampl e 57 | 0.02 | 0.01 | 0.01 | 0.03 | 0.01 |
| Exampl e 58 | 0.01 | 0.01 | 0.03 | 0.05 | 0.02 |
| Exampl e 59 | 0.007 | 0.007 | 0.01 | 0.02 | 0.01 |
| Exampl e 60 | 0.02 | 0.02 | 0.05 | 0.07 | 0.03 |

As can be seen from Table 18, the stock wet wipe compositions according to Examples 54 to 60 effectively controlled all of bacteria (*E. coli, S. Aureus, P. Aeruginosa*), filamentous fungi (*A. niger*), and yeasts (*C. albicans*) used for the antimicrobial tests.

The following Table 19 shows the results of antimicrobial tests against bacteria, filamentous fungi, and yeasts of the stock wet wipe compositions of Examples 61 to 64.

**[Table 19]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 61 | 0.01 | 0.007 | 0.02 | 0.05 | 0.01 |
| Exampl e 62 | 0.007 | 0.003 | 0.007 | 0.005 | 0.007 |
| Exampl e 63 | 0.01 | 0.01 | 0.02 | 0.05 | 0.02 |
| Exampl e 64 | 0.005 | 0.001 | 0.002 | 0.005 | 0.001 |

As can be seen from Table 19, the stock wet wipe compositions according to Examples 61 to 64 effectively controlled all of bacteria (*E. coli, S. Aureus, P. Aeruginosa*), filamentous fungi (*A. niger*), and yeasts (*C. albicans*) used for the antimicrobial tests.

The following Table 20 shows the results of antimicrobial tests against bacteria, filamentous fungi, and yeasts of the stock wet wipe compositions of Examples 65 to 69.

**[Table 20]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 65 | 0.005 | 0.005 | 0.005 | 0.05 | 0.03 |
| Exampl e 66 | 0.007 | 0.005 | 0.02 | 0.05 | 0.03 |
| Exampl e 67 | 0.005 | 0.005 | 0.01 | 0.02 | 0.01 |
| Exampl e 68 | 0.007 | 0.005 | 0.03 | 0.05 | 0.03 |
| Exampl e 69 | 0.007 | 0.005 | 0.02 | 0.05 | 0.03 |

As can be seen from Table 20, the stock wet wipe compositions according to Examples 65 to 69 effectively controlled all of bacteria (*E. coli, S. Aureus, P. Aeruginosa*), filamentous fungi (*A. niger*), and yeasts (*C. albicans*) used for the antimicrobial tests.

### [Tables 21 to 24] Antimicrobial test results of stock shampoo compositions

The following Table 21 shows the results of the antimicrobial tests against bacteria, filamentous fungi, and yeasts of the stock shampoo compositions of Examples 70 to 76.

**[Table 21]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 70 | 0.03 | 0.03 | 0.05 | 0.03 | 0.01 |
| Exampl e 71 | 0.02 | 0.01 | 0.01 | 0.01 | 0.01 |
| Exampl e 72 | 0.01 | 0.007 | 0.007 | 0.01 | 0.007 |
| Exampl e 73 | 0.02 | 0.02 | 0.02 | 0.05 | 0.01 |
| Exampl e 74 | 0.02 | 0.01 | 0.02 | 0.05 | 0.01 |
| Exampl e 75 | 0.01 | 0.007 | 0.02 | 0.05 | 0.01 |
| Exampl e 76 | 0.01 | 0.01 | 0.01 | 0.01 | 0.01 |

As can be seen from Table 21, the stock shampoo compositions according to Examples 70 to 76 effectively controlled all of bacteria (*E. coli, S. Aureus, P. Aeruginosa*), filamentous fungi (*A. niger*), and yeasts (*C. albicans*) used for the antimicrobial tests.

The following Table 22 shows the results of antimicrobial tests against bacteria, filamentous fungi, and yeasts of the stock shampoo compositions of Examples 77 to 83.

**[Table 22]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 77 | 0.01 | 0.01 | 0.01 | 0.02 | 0.01 |
| Exampl e 78 | 0.005 | 0.002 | 0.005 | 0.002 | 0.002 |
| Exampl e 79 | 0.01 | 0.007 | 0.02 | 0.05 | 0.007 |
| Exampl e 80 | 0.02 | 0.01 | 0.01 | 0.03 | 0.01 |
| Exampl e 81 | 0.01 | 0.01 | 0.03 | 0.05 | 0.01 |
| Exampl e 82 | 0.007 | 0.005 | 0.007 | 0.01 | 0.01 |
| Exampl e 83 | 0.01 | 0.01 | 0.03 | 0.05 | 0.01 |

As can be seen from Table 22, the stock shampoo compositions according to Examples 77 to 83 effectively controlled all of bacteria (*E. coli, S. Aureus, P. Aeruginosa*), filamentous fungi (*A. niger*), and yeasts (*C. albicans*) used for the antimicrobial tests.

The following Table 23 shows the results of antimicrobial tests against bacteria, filamentous fungi, and yeasts of the stock shampoo compositions of Examples 84 to 87.

**[Table 23]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 84 | 0.005 | 0.005 | 0.02 | 0.03 | 0.007 |
| Exampl e 85 | 0.005 | 0.003 | 0.005 | 0.003 | 0.005 |
| Exampl e 86 | 0.01 | 0.007 | 0.01 | 0.05 | 0.01 |
| Exampl e 87 | 0.005 | 0.0007 | 0.003 | 0.003 | 0.001 |

As can be seen from Table 23, the stock shampoo compositions according to Examples 84 to 87 effectively controlled all of bacteria (*E. coli, S. Aureus, P. Aeruginosa*), filamentous fungi (*A. niger*), and yeasts (*C. albicans*) used for the antimicrobial tests.

The following Table 24 shows the results of antimicrobial tests against bacteria, filamentous fungi, and yeasts of the stock shampoo compositions of Examples 88 to 92.

**[Table 24]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 88 | 0.003 | 0.003 | 0.005 | 0.02 | 0.01 |
| Exampl e 89 | 0.005 | 0.005 | 0.01 | 0.03 | 0.02 |
| Exampl e 90 | 0.003 | 0.003 | 0.007 | 0.01 | 0.01 |
| Exampl e 91 | 0.005 | 0.005 | 0.01 | 0.02 | 0.02 |
| Exampl e 92 | 0.005 | 0.005 | 0.007 | 0.02 | 0.02 |

As can be seen from Table 24, the stock shampoo compositions according to Examples 88 to 92 effectively controlled all of bacteria (*E. coli, S. Aureus, P. Aeruginosa*), filamentous fungi (*A. niger),* and yeasts (*C. albicans*) used for the antimicrobial tests.

### [Tables 25 to 28] Antimicrobial test results of stock cosmetic cream compositions

The following Table 25 shows the results of the antimicrobial tests against bacteria, filamentous fungi, and yeasts of the stock cosmetic cream compositions of Examples 93 to 99.

**[Table 25]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Example 93 | 0.07 | 0.03 | 0.1 | 0.03 | 0.01 |
| Example 94 | 0.01 | 0.01 | 0.02 | 0.02 | 0.01 |
| Example 95 | 0.01 | 0.007 | 0.01 | 0.01 | 0.007 |
| Example 96 | 0.03 | 0.02 | 0.03 | 0.05 | 0.01 |
| Example 97 | 0.02 | 0.02 | 0.02 | 0.05 | 0.01 |
| Example 98 | 0.02 | 0.01 | 0.02 | 0.05 | 0.01 |
| Example 99 | 0.01 | 0.01 | 0.01 | 0.02 | 0.01 |

As can be seen from Table 25, the stock cosmetic cream compositions according to Examples 93 to 99 effectively controlled all of bacteria (*E. coli, S. Aureus, P. Aeruginosa*), filamentous fungi (*A. niger*), and yeasts (*C. albicans*) used for the antimicrobial tests.

The following Table 26 shows the results of antimicrobial tests against bacteria, filamentous fungi, and yeasts of the stock cosmetic cream compositions of Examples 100 to 106.

**[Table 26]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 100 | 0.01 | 0.01 | 0.02 | 0.03 | 0.01 |
| Exampl e 101 | 0.007 | 0.005 | 0.007 | 0.01 | 0.007 |
| Exampl e 102 | 0.01 | 0.01 | 0.02 | 0.05 | 0.01 |
| Exampl e 103 | 0.01 | 0.01 | 0.01 | 0.02 | 0.01 |
| Exampl e 104 | 0.01 | 0.007 | 0.03 | 0.05 | 0.01 |
| Exampl e 105 | 0.007 | 0.007 | 0.01 | 0.01 | 0.007 |
| Exampl e 106 | 0.02 | 0.02 | 0.03 | 0.05 | 0.02 |

As can be seen from Table 26, the stock cosmetic cream compositions according to Examples 100 to 106 effectively controlled all of bacteria (*E. coli, S. Aureus, P. Aeruginosa*), filamentous fungi (*A*. *niger),* and yeasts (*C. albicans*) used for the antimicrobial tests.

The following Table 27 shows the results of antimicrobial tests against bacteria, filamentous fungi, and yeasts of the stock cosmetic cream compositions of Examples 107 to 110.

**[Table 27]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 107 | 0.01 | 0.007 | 0.03 | 0.05 | 0.01 |
| Exampl e 108 | 0.005 | 0.002 | 0.007 | 0.005 | 0.007 |
| Exampl e 109 | 0.02 | 0.01 | 0.02 | 0.05 | 0.01 |
| Exampl e 110 | 0.005 | 0.001 | 0.005 | 0.003 | 0.001 |

As can be seen from Table 27, the stock cosmetic cream compositions according to Examples 107 to 110 effectively controlled all of bacteria (*E. coli, S. Aureus, P. Aeruginosa*), filamentous fungi (*A. niger*), and yeasts (*C. albicans*) used for the antimicrobial tests.

The following Table 28 shows the results of antimicrobial tests against bacteria, filamentous fungi, and yeasts of the stock cosmetic cream compositions of Examples 111 to 115.

**[Table 28]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 111 | 0.01 | 0.005 | 0.01 | 0.05 | 0.03 |
| Exampl e 112 | 0.005 | 0.005 | 0.01 | 0.05 | 0.02 |
| Exampl e 113 | 0.007 | 0.005 | 0.02 | 0.03 | 0.01 |
| Exampl e 114 | 0.007 | 0.007 | 0.01 | 0.05 | 0.05 |
| Exampl e 115 | 0.007 | 0.007 | 0.015 | 0.05 | 0.05 |

As can be seen from Table 28, the stock cosmetic cream compositions according to Examples 111 to 115 effectively controlled all of bacteria (*E. coli, S. Aureus, P. Aeruginosa*), filamentous fungi (*A. niger*), and yeasts (*C. albicans*) used for the antimicrobial tests.

### [Tables 29 to 32] Antimicrobial test results of cosmetic pack compositions

The following Table 29 shows the results of the antimicrobial tests against bacteria, filamentous fungi, and yeasts of the cosmetic pack compositions of Examples 116 to 122.

**[Table 29]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 116 | 0.01 | 0.01 | 0.02 | 0.03 | 0.01 |
| Exampl e 117 | 0.007 | 0.007 | 0.01 | 0.01 | 0.007 |
| Exampl e 118 | 0.005 | 0.005 | 0.005 | 0.007 | 0.005 |
| Exampl e 119 | 0.02 | 0.01 | 0.02 | 0.05 | 0.01 |
| Exampl e 120 | 0.01 | 0.01 | 0.01 | 0.03 | 0.01 |
| Exampl e 121 | 0.01 | 0.007 | 0.01 | 0.03 | 0.01 |
| Exampl e 122 | 0.007 | 0.007 | 0.01 | 0.01 | 0.007 |

As can be seen from Table 29, the cosmetic pack compositions according to Examples 116 to 122 effectively controlled all of bacteria (*E. coli, S. Aureus, P. Aeruginosa*), filamentous fungi (*A. niger*), and yeasts (*C*. *albicans*) used for the antimicrobial tests.

The following Table 30 shows the results of antimicrobial tests against bacteria, filamentous fungi, and yeasts of the cosmetic pack compositions of Examples 123 to 129.

**[Table 30]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 123 | 0.01 | 0.007 | 0.007 | 0.01 | 0.01 |
| Exampl e 124 | 0.003 | 0.002 | 0.005 | 0.005 | 0.002 |
| Exampl e 125 | 0.01 | 0.007 | 0.02 | 0.03 | 0.01 |
| Exampl e 126 | 0.01 | 0.01 | 0.007 | 0.01 | 0.005 |
| Exampl e 127 | 0.007 | 0.007 | 0.03 | 0.03 | 0.01 |
| Exampl e 128 | 0.005 | 0.002 | 0.005 | 0.01 | 0.007 |
| Exampl e 129 | 0.02 | 0.01 | 0.02 | 0.03 | 0.01 |

As can be seen from Table 30, the cosmetic pack compositions according to Examples 123 to 129 effectively controlled all of bacteria (*E. coli, S. Aureus, P. Aeruginosa*), filamentous fungi (*A. niger*), and yeasts (*C*. *albicans*) used for the antimicrobial tests.

The following Table 31 shows the results of antimicrobial tests against bacteria, filamentous fungi, and yeasts of the cosmetic pack compositions of Examples 130 to 133.

**[Table 31]**

| (%) | Bacteria | | | Flamentou s fungi (molds) | Yeasts |
|---|---|---|---|---|---|
| | *E. col i* | *S.Aureu s* | *P.Aeruginos a* | *A.niger* | *C.albican s* |
| Exampl e 130 | 0.007 | 0.005 | 0.01 | 0.03 | 0.007 |
| Exampl e 131 | 0.003 | 0.001 | 0.005 | 0.003 | 0.005 |
| Exampl e 132 | 0.01 | 0.007 | 0.01 | 0.03 | 0.01 |
| Exampl e 133 | 0.003 | 0.0007 | 0.002 | 0.002 | 0.0007 |

As can be seen from Table 31, the cosmetic pack compositions according to Examples 130 to 133 effectively controlled all of bacteria (*E. coli, S. Aureus, P. Aeruginosa*), filamentous fungi (*A. niger*), and yeasts (*C*. *albicans*) used for the antimicrobial tests.

The following Table 32 shows the results of antimicrobial tests against bacteria, filamentous fungi, and yeasts of the cosmetic pack compositions of Examples 134 to 138.

**[Table 32]**

| (%) | Bacteria | | | Flamentou s fungi (molds) | Yeasts |
|---|---|---|---|---|---|
| | *E.col i* | *S.Aureu s* | *P.Aeruginos a* | *A.niger* | *C.albican s* |
| Exampl e 134 | 0.01 | 0.07 | 0.01 | 0.04 | 0.01 |
| Exampl e 135 | 0.007 | 0.003 | 0.007 | 0.05 | 0.02 |
| Exampl e 136 | 0.005 | 0.003 | 0.007 | 0.03 | 0.01 |
| Exampl e 137 | 0.01 | 0.007 | 0.01 | 0.05 | 0.03 |
| Exampl e 138 | 0.01 | 0.007 | 0.015 | 0.05 | 0.03 |

As can be seen from Table 32, the cosmetic pack compositions according to Examples 134 to 138 effectively controlled all of bacteria (*E. coli, S. Aureus, P. Aeruginosa*), filamentous fungi (*A. niger*), and yeasts (*C*. *albicans*) used for the antimicrobial tests.

## Claims

1. An antimicrobial and preservative composition comprising:
(a) a compound represented by Formula 1 below; and
(b) a compound represented by Formula 2, Formula 3, Formula 4 or Formula 5 below: 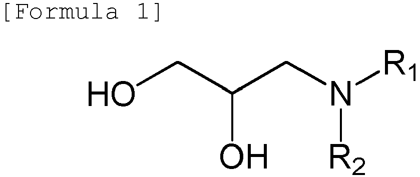 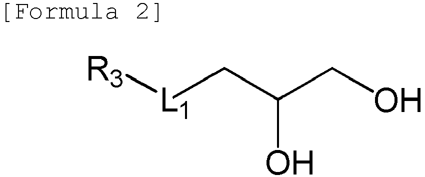 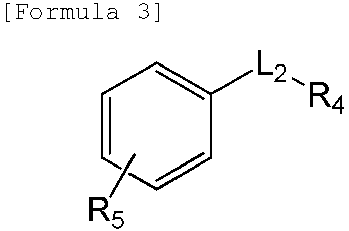 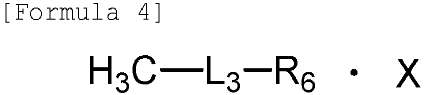 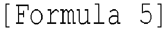 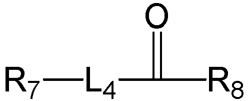 wherein
in Formula 1,
R₁ is hydrogen, and
R₂ is substituted or unsubstituted C₆-C₁₈ alkyl, or substituted or unsubstituted C₆-C₁₈ alkenyl,
in Formula 2,
L₁ is -CH₂-, -O-, -C(=O)- or -O-C(=O)-, and
R₃ is substituted or unsubstituted C₁-C₁₅ alkyl, or substituted or unsubstituted C₂-C₁₅ alkenyl,
in Formula 3,
L₂ is -(CH=CH)-, -(CH₂)ₘ-, -C=O- or -O-,
R₄ is -C(=O)-Rₐ, -OH, -O⁻Na⁺, -(CH₂)ₙ-OH, substituted or unsubstituted C₁-C₁₅ alkyl, or substituted or unsubstituted C₂-C₁₅ alkenyl,
R₅ is hydrogen, halogen, -OH, -OR_{b} or -(C=O)-O-R_{c}, and
Rₐ to R_{c} are each independently hydrogen, substituted or unsubstituted C₁-C₁₅ alkyl, or substituted or unsubstituted C₂-C₁₅ alkenyl,
wherein m and n are each independently an integer of 1 to 5,
in Formula 4,
L₃ is -C(=O)-, -(CH=CH)-(CH₂)ₒ-, -C(CH₃)₂-CH₂-CH(CH₃)-CH₂- or -(CH₂)ₚ-,
wherein o and p are each independently an integer of 1 to 15, and
-(CH₂)ₚ- is substituted with C₁-C₁₅ alkyl,
R₆ is 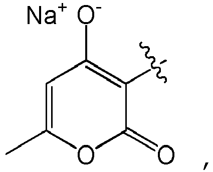 -C(=O)-NH-OH, 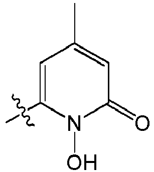 or 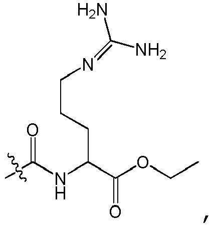
X is NH₂-CH₂-CH₂-OH or nothing (null), and
in Formula 5,
R₇ is substituted or unsubstituted C₁-C₁₅ alkyl, -COOH, -OR_{d} or -COO⁻Na⁺,
R₈ is substituted or unsubstituted C₁-C₁₅ alkyl, -OH or -O⁻Na⁺,
L₄ is substituted or unsubstituted -(CH₂)_{q}-, substituted or unsubstituted -(CH₂)ᵣ-N(Rₑ)-(CH₂)ₛ-, or substituted or unsubstituted phenylene(-C₆H₄-),
R_{d} is substituted or unsubstituted C₁-C₁₅ alkyl, and
Rₑ is substituted or unsubstituted C₁-C₁₅ alkyl, or - (CH₂)ₜ-N[(CH₂)ᵤ-CO₂⁻)]₂,
wherein q, r, s, t, and u are each independently an integer of 1 to 5.

2. The antimicrobial and preservative composition according to claim 1, wherein the antimicrobial and preservative composition has antibacterial or antifungal activity against bacteria or fungi.

3. The antimicrobial and preservative composition according to claim 2, wherein the antimicrobial and preservative composition has antibacterial activity against *Escherichia coli, Pseudomonas aeruginosa, Staphylococcus aureus, Klebsiella pneumoniae,* MRSA (*methicillin-resistant Staphylococcus aureus*), *Bacillus subtilis, Vibrio parahaemolyticus, Salmonella typhimurium, Listeria monocytogenes* and *Shigella flexneri,* and has antifungal activity against *Candida albicans,* and *Aspergillus niger.*

4. The antimicrobial and preservative composition according to claim 1, wherein the compound represented by Formula 1 and the compound represented by Formula 2, Formula 3, Formula 4 or Formula 5 are present in a weight ratio of 0.01:9.99 to 9.99:0.01.

5. The antimicrobial and preservative composition according to claim 1, wherein the compound represented by Formula 1 is 3-(dodecylamino)propane-1,2-diol represented by the following Formula 1-1, or 3-(tetradecylamino)propane-1,2-diol represented by the following Formula 1-2:

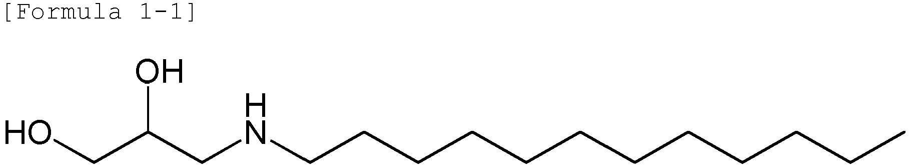

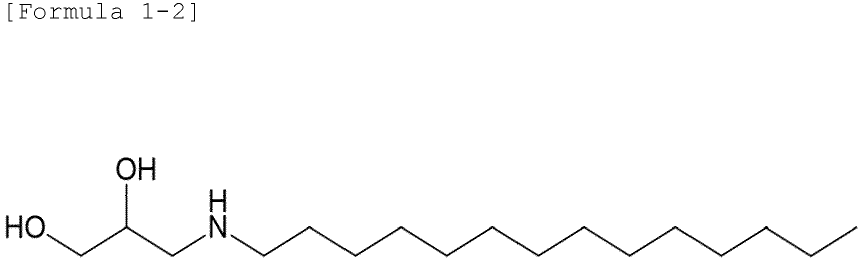

6. The antimicrobial and preservative composition according to claim 1, wherein, in the compound represented by Formula 2, L₁ is -CH₂-, -O-, or -O-C(=O)-, and R₃ is substituted or unsubstituted C₂-C₁₀ alkyl.

7. The antimicrobial and preservative composition according to claim 1, wherein the compound represented by Formula 2 comprises at least one selected from the group consisting of hexane-1,2-diol, octane-1,2-diol, decane-1,2-diol, hexyl glycerin, octyl glycerin, ethylhexylglycerin, and glyceryl monocaprylate.

8. The antimicrobial and preservative composition according to claim 1, wherein in the compound represented by Formula 3,
L₂ is -(CH=CH)-, -(CH₂)ₘ-, -C=O- or -O-,
R₄ is -C(=O)-Rₐ, -OH, -O⁻Na⁺, -(CH₂)ₙ-OH, or substituted or unsubstituted C₁-C₆ alkyl,
R₅ is hydrogen, -OH or -OR_{b},
Rₐ and R_{b} are each independently hydrogen, substituted or unsubstituted C₁-C₆ alkyl, and
m and n are each independently an integer of 2 to 4.

9. The antimicrobial and preservative composition according to claim 1, wherein the compound represented by Formula 3 comprises at least one selected from the group consisting of cinnamaldehyde, 2-methoxycinnamaldehyde, raspberry ketone, 3-phenyl-1-propanol, 4-hydroxy acetophenone, sodium benzoate, and phenoxy EtOH.

10. The antimicrobial and preservative composition according to claim 1, wherein in the compound represented by Formula 4,
L₃ is -C(=O)-, -C(CH₃)₂-CH₂-CH(CH₃)-CH₂- or -(CH₂)ₚ-,
p is an integer of 5 to 12,
R₆ is 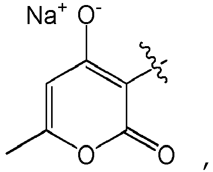 -C(=O)-NH-OH, 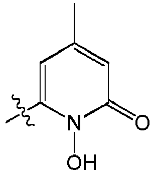 or 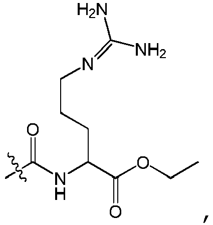 and
X is NH₂-CH₂-CH₂-OH or nothing (null) .

11. The antimicrobial and preservative composition according to claim 1, wherein the compound represented by Formula 4 comprises at least one selected from the group consisting of sodium dehydroacetate, caprylhydroxamic acid, piroctone olamine, and lauroyl arginine ethyl ester.

12. The antimicrobial and preservative composition according to claim 1, wherein in the compound represented by Formula 5,
R₇ is -COOH, -OR_{d} or -COO⁻Na⁺,
R₈ is unsubstituted C₁-C₃ alkyl, -OH or -O⁻Na⁺,
L₄ is -(CH₂)_{q}- substituted or unsubstituted with at least one substituent selected from the group consisting of hydroxy, unsubstituted C₁-C₃ alkyl and carboxyl, substituted or unsubstituted -(CH₂)ᵣ-N(Rₑ)-(CH₂)ₛ-, or unsubstituted phenylene (-C₆H₄-),
R_{d} is unsubstituted C₁-C₃ alkyl,
Rₑ is -(CH₂)ₜ-N[(CH₂)ᵤ-CO₂⁻)]₂, and
q, r, s, t, and u are each independently an integer of 1 to 3.

13. The antimicrobial and preservative composition according to claim 1, wherein the compound represented by Formula 5 comprises at least one selected from the group consisting of levulinic acid, citric acid, sodium anisate, disodium EDTA, and tetrasodium EDTA.

14. A product comprising the antimicrobial and preservative composition according to claim 1.

15. The product according to claim 14, wherein the product is a cosmetic, hygiene product or household chemical product.

16. The product according to claim 15, wherein the cosmetic is formulated into at least one selected from the group consisting of solutions, suspensions, emulsions, pastes, gels, creams, pacts, powders, emulsion foundations, wax foundations, sprays, soaps, face cleansers, body cleansers, hair shampoos and hair rinses.

17. The product according to claim 15, wherein the sanitary product is selected from the group consisting of dishwashing detergents, dishwasher detergents, and wet wipe.

18. The product according to claim 15, wherein the household chemical product is selected from the group consisting of laundry detergents, residential cleaners, deodorants, air cleaners, indoor air fresheners, fragrances, fabric softeners, multi-purpose cleaners, disinfectants, antimicrobials, sanitizers, and fungistats.

19. The product according to claim 15, wherein the antimicrobial and preservative composition is present in an amount of 0.01 to 10% by weight with respect to the weight of the cosmetic, sanitary or household chemical product.

20. An antibacterial method comprising treating bacteria with the antimicrobial and preservative composition according to claim 1.
